# EUROPEAN PATENT SPECIFICATION

(11) **EP 3 561 059 B1**
(45) Date of publication and mention of the grant of the patent: **09.10.2024**
(21) Application number: 17885347.9
(22) Date of filing: 22.12.2017
(51) Int. Cl.: C12N 15/10, C12N 15/85, C12N 15/877, C12N 9/16, C12N 9/78, C12N 9/02, C07K 14/47

(54) **COMPOSITION FOR BASE EDITING FOR ANIMAL EMBRYO AND BASE EDITING METHOD**
ZUSAMMENSETZUNG ZUR BASENEDITIERUNG FÜR TIEREMBRYO UND BASENEDITIERVERFAHREN
COMPOSITION POUR L'ÉDITION DE BASES POUR UN EMBRYON ANIMAL ET PROCÉDÉ D'ÉDITION DE BASES

(30) Priority: 23.12.2016 KR 20160178429
(43) Date of publication of application: 30.10.2019
(73) Proprietor: Institute for Basic Science, Daejeon 34047 (KR)
(72) Inventor: KIM, Jin-Soo, Seoul 08831 (KR); KIM, Kyoung Mi, Seoul 03024 (KR); RYU, Seuk Min, Seoul 08735 (KR)
(74) Representative: Reddie & Grose LLP
(86) International application number: PCT/KR2017/015380
(87) International publication number: WO 2018/117746

(56) References cited:
- EP-A1- 3 604 532
- WO-A2-2016/022363
- KR-A- 20150 101 477
- US-A1- 2015 166 980
- ALEXIS C. KOMOR ET AL: "Programmable editing of a target base in genomic DNA without double-stranded DNA cleavage", NATURE, vol. 533, no. 7603, 20 April 2016 (2016-04-20), London, pages 420 - 424, XP055551781, ISSN: 0028-0836, DOI: 10.1038/nature17946
- JUNHO K HUR ET AL: "Targeted mutagenesis in mice by electroporation of Cpf1 ribonucleoproteins", NATURE BIOTECHNOLOGY, vol. 34, no. 8, 6 June 2016 (2016-06-06), us, pages 807 - 808, XP055465092, ISSN: 1087-0156, DOI: 10.1038/nbt.3596
- "Meet the Editors", CELL, ELSEVIER, AMSTERDAM NL, vol. 165, no. 6, 2 June 2016 (2016-06-02), pages 12951297, XP029567391, ISSN: 0092-8674, DOI: 10.1016/J.CELL.2016.05.067
- JINGYING LI ET AL: "Generation of Targeted Point Mutations in Rice by a Modified CRISPR/Cas9 System", MOLECULAR PLANT, vol. 10, 8 December 2016 (2016-12-08), pages 526 - 529, XP055383084, DOI: http://dx.doi.org/10.1016/j.molp.2016.12.001
- KOMOR, ALEXIS C.: "Programmable editing of a target base in genomic DNA without double-stranded DNA cleavage", NATURE, 19 May 2016 (2016-05-19), pages 420 - 424, XP055548777, DOI: doi:10.1038/nature17946
- MA, YUNQING: "Targeted AID-mediated mutagenesis (TAM) enables efficient genomic diversification in mammalian cells", NATURE METHODS, vol. 13, no. 12, 10 October 2016 (2016-10-10), pages 1029 - 1035, XP055449587
- KIM, KYOUNGMI: "Highly efficient RNA-guided base editing in mouse embryos", NATURE BIOTECHNOLOGY, vol. 35, no. 5, 27 February 2017 (2017-02-27), pages 435 - 437, XP055479394, ISSN: 1087-0156, DOI: 10.1038/nbt.3816

## Description

### Technical Field

Provided are a base-editing composition comprising a deaminase and a target-specific nuclease and a base-editing method using the base-editing composition.

### Background Art

Most human genetic diseases are caused by single base substitution or point mutation rather than some insertions/deletions (indels) or broad chromosomal rearrangement in the genome. Genome editing mediated by programmable nuclease such as clustered, regular interspaced, short palindromic repeat (CRISPR)-Cas9 or Cpf1 system enables gene correction for genetic defects provoking genetic disease, but has a technical difficulty in inducing single base substitution in a target specific manner. This is because most of DNA double-stranded breaks (DSB) generated by programmable nucleases are repaired by error-prone non-homologous end-joining (NHEJ) other than homologous recombination (HR) using a template donor DNA. As a result, indels at nuclease targeted sites occur more frequently than single nucleotide substitutions.

Recently, it has been reported that Cas9 nickase (nCas9) or catalytically-deficient Cas9 (dCas9) linked to cytidine deaminase such as apolipoprotein B editing complex 1 (APOBEC1) or activation-induced deaminase (AID) substitutes C for T (or G for A) at target sites without producing DSBs. Such reports exhibit base editing in yeasts and cultured mammalian cells. These RNA-guided programmable deaminases expand the coverage of genome editing to another level and may suggest a method for inducing target mutations or conducting gene editing across all organisms including humans. However, it has to be proved that RNA-programmable deaminases have a base editing function *in vivo.*

### Detailed Description of the Invention

### Technical Problem

The disclosure provides a technique for inducing a single nucleotide substitution in eukaryotic cells, such as murine cells (e.g., animal cells including mammalian cells but excluding human embryos by using a programmable deaminase.

An embodiment provides a base editing composition comprising: (1) a deaminase or a coding gene therefor, and (2) a target-specific nuclease or a coding gene therefor. The cell may be a eukaryotic cell (e.g., a cell from a eukaryotic animal), and the base editing method may be to perform base editing (e.g., base substitution) in a eukaryotic cell.

The target-specific nuclease may include: an RNA-guided nuclease; and a guide RNA hybridizable with a target site in a target gene (having a complementary nucleotide sequence) or a coding DNA therefor (or a recombinant vector carrying the coding DNA). In this regard, the base editing composition may comprise: (1) a deaminase or a coding gene therefor (mRNA or a recombinant vector carrying the coding DNA), (2) an RNA-guided nuclease or a coding gene therefor (mRNA or a recombinant vector carrying the coding DNA), and (3) a guide RNA or a coding gene (DNA) therefor.

The base editing composition may further comprise: (4) an uracil DNA glycosylase inhibitor (UGI) or a coding gene therefor; and/or (5) a nuclear localization sequence (NLS) or a coding gene therefor in addition to (1) a deaminase or a coding gene therefor (mRNA or a recombinant vector carrying the coding DNA), (2) an RNA-guided nuclease or a coding gene therefor (mRNA or a recombinant vector carrying the coding DNA), and (3) a guide RNA or a coding gene therefor (DNA). In the case where the base editing composition employs a fusion protein or gene form in which the deaminase, the RNA-guided nuclease, and optionally UGI and/or NLS or coding genes therefor are linked, respectively, a suitable linker may be given between one of the adjacent coupled proteins or genes, for example, between the deaminase and the RNA-guided nuclease, between the nuclease and UGI, and between UGI and NLS (a peptide linker (3-30 or 3-20 a.a.) for the fusion protein and an oligonucleotide linker (9-90 or 9-60 nt) for the fusion gene).

Another embodiment provides a base editing method comprising a step of introducing the base editing composition into a cell. The cell may be a eukaryotic cell and the base editing method may be to conduct editing (for example, base substitution) in a eukaryotic cell.

According to the invention there is provided a base editing method comprising: directly injecting 1) a ribonucleoprotein complex or mixture, the ribonucleoprotein complex or mixture comprising a cytidine deaminase, an RNA-guided nuclease, and a guide RNA , or 2) a mixture of a cytidine deaminase-encoding mRNA, an RNA-guided 10 nuclease-encoding mRNA, and a guide RNA into the non-human mammalian embryonic cell without using a recombinant vector. As stated supra, the base editing composition used in the introducing step conducted through ¹) or ²) may further comprise a uracil DNA glycosylase inhibitor (UGI) or a coding gene therefor and/or (5) a nuclear localization sequence (NLS) or a coding gene therefor, and optionally a suitable linker.

### Technical Solution

The present inventors successfully induced single nucleotide substitutions in eukaryotic cells such as murine cells (e.g., animal cells such as mammalian cells, etc.) by using a programmable deaminase.

An embodiment provides a base editing composition comprising: (1) a deaminase or a coding gene therefor, and (2) a target-specific nuclease or a coding gene therefor. The base editing composition may have base editing (e.g., base substitution) activity in eukaryotic cells. The eukaryotic cells may be cells of eukaryotic animals, for example, non-human embryonic cells. In one embodiment, the eukaryotic cells may be non-human mammalian cells, for example, mammalian embryonic cells.

As used herein, the term "coding gene" is intended to encompass cDNA, rDNA, a recombinant vector carrying the same, and mRNA.

As used herein, the "deaminase" is a generic term for enzymes having the activity of removing an amine group from certain bases in eukaryotic cells and may be, for example, cytidine deaminase, which converts cytidine to uridine, and/or adenosine deaminase. In one embodiment, the deaminase may be at least one selected from the group consisting of apolipoprotein B editing complex 1 (APOBEC1), activation-induced deaminase (AID), tRNA-specific adenosine deaminase (tadA), and the like, but is not limited thereto. Single nucleotide substitution in eukaryotic cells can be induced by such base conversion (for example, conversion of cytidine to uridine).

The target-specific nuclease may include an RNA-guided nuclease and a guide RNA capable of hybridizing with (or having a complementary sequence to) a target site of a target gene or a coding DNA therefor (or a recombinant vector carrying the coding DNA). In this context, the base editing composition may comprise: (1) a deaminase or a coding gene therefor (mRNA or a recombinant vector carrying the coding DNA), (2) a modified RNA-guided nuclease or a coding gene therefor (mRNA or a recombinant vector carrying the coding DNA), and (3) a guide RNA or a coding DNA therefor.

In an embodiment, the base editing composition may further comprise: (4) a uracil DNA glycosylase inhibitor (UGI) or a coding gene therefor; and/or (5) nuclear localization sequence (NLS) or a coding gene therefor in addition to (1) a deaminase or a coding gene therefor (mRNA or a recombinant vector carrying the coding DNA), (2) an RNA-guided nuclease or a coding gene therefor (mRNA or a recombinant vector carrying the coding DNA), and (3) a guide RNA or a coding gene therefor (DNA). When the base editing composition employs a fusion protein or gene form in which the deaminase, the RNA-guided nuclease, and optionally UGI and/or NLS or coding genes therefor are linked, respectively, a suitable linker may be given between one of the adjacent coupled proteins or genes, for example, between the deaminase and the RNA-guided nuclease, between the nuclease and UGI, and between UGI and NLS (a peptide linker (3-30 or 3-20 a.a.) for the fusion protein and an oligonucleotide linker (9-90 or 9-60 nt) for the fusion gene).

In an embodiment, the RNA-guided nuclease may be a modified RNA-guided nuclease that is modified to lose the activity of forming DNA double-stranded breaks.

The modified RNA-guided nuclease may be a modified Cas9 (CRISPR associated protein 9) or modified Cpf1 (CRISPR from Prevotella and Francisella 1) system that is modified to cut one strand in a target gene (nick formation). In one embodiment, the modified RNA-guided nuclease may be selected from the group consisting of Cas9 nickase (nCas9) and catalytically-deficient Cas9 (dCas9).

When the base editing composition comprises a deaminase-encoding gene and an RNA-guided nuclease-encoding gene, the encoding gene may be DNA or mRNA. In addition, the deaminase-encoding gene and the RNA-guided nuclease-encoding gene may be included in a form of mRNA or recombinant vectors that carry the DNAs respectively (i.e., one recombinant vector carrying the 5 deaminase-encoding DNA and one recombinant vector carrying the RNA guide nuclease-encoding DNA) or a recombinant vector that carries the DNAs together. The guide RNA may be CRISPR RNA (crRNA), trans-activating crRNA (tracrRNA), double guide RNA including crRNA and tracrRNA (a complex of crRNA and tracrRNA), or single guide RNA (sgRNA). In an embodiment, the base editing composition may comprise mRNAs coding for a deaminase and a modified RNA-guided nuclease, and a guide RNA, or a ribonucleoprotein (RNP) inclusive of a deaminase, a modified RNA-guided nuclease, and a guide RNA. The ribonucleoprotein may include a deaminase, a modified RNA-guided nuclease, and a guide RNA in mixture or may exist as a complex in which a deaminase, a modified RNA-guided nuclease, and a guide RNA are associated.

Another embodiment provides a base editing method comprising a step of introducing the base editing composition into a cell. The cell may be a eukaryotic cell and the base editing method may be to conduct base editing (e.g., base substitution) in a eukaryotic cell.

The eukaryotic cell may be a eukaryotic animal cell, for example, a eukaryotic non-human embryonic cell. In an embodiment, the cell may be a mammalian cell, for example, a non-human mammalian embryonic cell. The base editing method may achieve a base conversion rate (base substitution rate) of 40% or higher, 45% or higher, 50% or higher, 55% or higher, 60% or higher, 65% or higher, 70% or higher, 75% or higher, 80% or higher, 85% or higher, 90% or higher, 95% or higher, 97% or higher, 99% or higher, or 100% in eukaryotic cells (e.g., eukaryotic non-human embryonic cells). In addition, the base editing method may cause various mutants by base substitution to create a stop codon within a gene (e.g., coding sequence) for gene knockout, to introduce mutations in non-coding DNA sequences that do not encode protein sequences, etc.

Particularly, the base editing composition may be applied to nonhuman mammalian embryos to effectively construct adult mammals having a gene desirably knocked out therein or a desirable mutation introduced thereto.

The step of introducing the base editing composition into a cell may be a step of introducing a deaminase or a deaminase-encoding gene, an RNA-guided nuclease or an RNA-guided nuclease-encoding gene, and a guide RNA or a guide RNA-encoding gene to a cell. Of the coding genes, at least one may be included in respective or one recombinant vector for use in introduction.

In one embodiment, the step of introducing the base editing composition into a cell may be conducted by
1) directly injecting a cytidine deaminase, an RNA-guided nuclease, and a guide RNA (for example, in a mixture form or in a ribonucleic acid protein form in which a cytidine deaminase, a RNA-guided nuclease, and a guide RNA are complexed) into the cell, or
2) directly injecting a cytidine deaminase-encoding mRNA, an RNA-guided nuclease-encoding mRNA, and a guide RNA in mixture or individually into the cell. As used herein, the term "directly injecting" means that the cytidine deaminase, the RNA-guided nuclease, and the guide RNA of 2) (e.g., the cytidine deaminase, the RNA-guided nuclease, and the guide RNA in mixture or in the complex form of ribonucleoprotein), or the cytidine deaminase-encoding mRNA, RNA-guided nuclease-encoding mRNA and the guide RNA of 3) pass through the cell membrane and/or nuclear membrane and are transferred to the genome without using a recombinant vector, and may be conducted by electroporation, lipofection, microinjection, etc. In the disclosure, the cells to which the base editing composition is applicable may be eukaryotic cells, for example, cells from eukaryotic animals.

The eukaryotic animals may be mammals including primates, such as humans, (excluding human embryos) and rodents, such as mice, etc. The cells from eukaryotic animals may be non-human mammalian embryos. For example, the embryos may be taken from the oviduct of a superovulated female mammal (superovulated by injection of gonadal hormones, such as PMSG (Pregnant Mare Serum Gonadotropin), hCG (human Choirinic Gonadotropin), and the like) after crossing the superovulated female animals with male mammals. The non-human embryo to which the base editing composition is applied (injected) may be a one-cell zygote at fertilization.

As used herein, the term "base editing" means base mutation (substitution, deletion, or insertion) incurring point mutation at a target site within a target gene and can be distinguished from gene editing in terms of the scale of mutated bases, a small number of mutated bases (one or two bases, i.e., one base) in base editing and relatively many mutated bases in gene editing. The base editing may not result in double-stranded DNA cleavage.

As used herein, the term "base mutation (or base substitution)" means mutation (e.g., substitution) on a nucleotide inclusive of the corresponding base and may be used interchangeably with "nucleotide mutation (or nucleotide substitution)". The base mutation may occur on either or both of allele genes.

In one embodiment, the base mutation and the base editing resulting therefrom may be conducted in various manners including creating a stop codon or a codon accounting for a different amino acid from a wild-type amino acid on a target site to knock a target gene out, or introducing a mutation in a non-coding DNA sequence, which does not encode a protein sequence, etc., but is not limited thereto.

In the disclosure, the base editing or base mutation may be conducted *in vitro* or *in vivo,* where the *in vivo* method is not a method of treatment The term "base sequence", as used herein, means a nucleotide sequence including corresponding bases and can be used interchangeably with a nucleotide sequence or a nucleic acid sequence.

As used herein,
"target gene" means a gene as an object to which base editing (or base mutation) is applied,
"target site" or "target region" means a site or region at which a target-specific nuclease performs base editing in a target gene. In one embodiment, when the target-specific nuclease includes an RNA-guided nuclease (RNA-guided engineered nuclease; RGEN), the target site or target region is intended to be a gene site (a double strand, or any one single strand in a double strand) which is located adjacent to the 5'- and/or 3'-end of the RNA-guided nuclease-recognized sequence (PAM sequence) in a target gene and has a maximum length of about 50 bp or about 40 bp.

In one embodiment, when the target-specific nuclease includes an RNA-guided nuclease, a guide RNA containing a targeting sequence may be included together with the RNA-guided nuclease. The "targeting sequence" may be a guide RNA site including a base sequence complementary to (hybridizable with) a consecutive base sequence of about 15 to about 30 nucleotides (nt), about 15 to about 35 nt, about 17 to about 23 nt, or about 18 to about 22 nt, e.g., about 20 nt on a target site. The base sequence on a target site, complementary to the targeting sequence, is called a "target sequence". The "target sequence" may mean a consecutive base sequence of about 15 nt to about 30 nt, about 15 nt to about 25 nt, about 17 nt to about 23 nt, or about 18 nt to about 22 nt, for example, about 20 nt located adjacent to the 5'- and/or 3'-end of a PAM sequence recognized by an RNA-guided nuclease.

A deaminase is a generic name for enzymes having the activity of removing an amine group from certain bases in eukaryotic cells, as exemplified by cytidine deaminase and/or adenosine deaminase that converts cytidine to uridine. In one embodiment, the deaminase may be at least one selected from the group consisting of APOBEC (apolipoprotein B mRNA editing enzyme, catalytic polypeptide-like), AID (activation-induced deaminase), and tadA (tRNA-specific adenosine deaminase), but is not limited thereto. APOBEC1, AID, and tadA may be derived from prokaryotic animals such as E. coli, or eukaryotic animals, such as mammals, for example, primates including humans, rodents including mice, etc.

In one embodiment, the APOBEC may be at least one selected from the group consisting of APOBEC1 (apolipoprotein B editing complex 1), APOBEC2, APOBEC3B, APOBEC3C, APOBEC3D, APOBEC3E, APOBEC3F, APOBEC3G, APOBEC3H, and APOBEC4, which are all derived from mammals, for example, humans, rats, mice, etc.

The APOBEC1 may be at least one selected from the group consisting of human APOBEC1 (e.g., GenBank Accession No. NP_005880.2 (coding gene: NM_005889.3), NP_001291495.1 (coding gene: NM_001304566.1), NP_001635.2 (coding gene: NM_001644.4), etc.), murine APOBEC1 (e.g., GenBank Accession No. NP_001127863.1 (coding gene: NM_001134391.1), NP_112436.1 (coding gene: NM_031159.3), etc.), and rat APOBEC1 (e.g., GenBank Accession No. NP_037039.1 (SEQ ID NO: 6) (coding gene: NM_012907.2), etc.) and is not limited thereto.

The AID may be selected from the group consisting of human AID (e.g., GenBank Accession No. NP_001317272.1 (coding gene: NM_001330343.1), NP_065712.1 (coding gene: NM_020661.3) etc.) and murine AID (e.g., GenBank Accession No. NP_033775.1 (coding gene: NM_009645.2), etc.), but is not limited thereto.

The tadA may be at least one selected from the group consisting of E. coli tadA (e.g., GenBank Accession No. NP_417054.2, YP_002408701.1, YP_002413581.3, etc.) and is not limited thereto.

By the base conversion (e.g., conversion from cytidine to uridine), single nucleotide substitution can be induced in eukaryotic cells.

The deaminase may be used in the form of a protein, a gene coding therefor (e.g., DNA or mRNA), or a recombinant vector carrying the gene.

As used herein, the target-specific nuclease, also called programmable nuclease, is a generic name for all the nucleases (endonucleases) that can recognize and cleave specific sites on genomic DNA (single strand nick or double strand cleavage).

For example, the target-specific nuclease may be at least one selected from all nucleases that can recognize specific sequences of target genes and have nucleotide cleavage activity to incur indel (insertion and/or deletion) in the target genes.

For example, the target-specific nuclease may include at least one selected from the group consisting of RGENs (RNA-guided engineered nucleases, e.g., Cas protein (i.e., Cas9, etc.), Cpf1, etc.) derived from the CRISPR system, which is a microbial immune system, but is not limited thereto.

The target-specific nuclease may recognize a specific base sequence on a genome in prokaryotic cells and/or animal and plant cells (e.g., eukaryotic cells) including human cells (excluding human embryos to incur a double strand break (DSB). Resulting from the cleavage of double strands of DNA, the double strand break may form a blunt end or a cohesive end. DSB can be effectively repaired by a homologous recombination or non-homologous end-joining (NHEJ) mechanism within cells, during which a desired mutation can be introduced to a target site.

In one embodiment, the target-specific nuclease may be at least one selected from the group consisting of nucleases (e.g., endonucleases) included in the type II and/or type V CRISPR system, such as Cas proteins (e.g., Cas9 protein (CRISPR (Clustered regularly interspaced short palindromic repeats) associated protein 9)), Cpf1 protein (CRISPR from Prevotella and Francisella 1), etc. In this regard, the target-specific nuclease further comprises a target DNA-specific guide RNA for guiding to a target site on a genomic DNA. The guide RNA may be an RNA transcribed *in vitro,* for example, RNA transcribed from double-stranded oligonucleotides or a plasmid template, but is not limited thereto. The target-specific nuclease may act in a ribonucleoprotein (RNP) form in which the nuclease is associated with guide RNA to form a ribonucleic acid-protein complex (RNA-Guided Engineered Nuclease), *in vitro* or after transfer to a body (cell).

The Cas protein, which is a main protein component in the CRISPR/Cas system, accounts for activated endonuclease or nickase activity.

The Cas protein or gene information may be obtained from a well-known database such as GenBank at the NCBI (National Center for Biotechnology Information). By way of example, the Cas protein may be at least one selected from the group consisting of:
a Cas protein derived from *Streptococcus* sp., e.g., *Streptococcus pyogenes,* for example, Cas9 protein (i.e., SwissProt Accession number Q99ZW2 (NP_269215.1));
a Cas protein derived from *Campylobactersp.,* e.g., *Campylobacter jejuni,* for example, Cas9 protein;
a Cas protein derived from *Streptococcus* sp., e.g., *Streptococcus thermophiles* or *Streptococcus aureus,* for example, Cas9 protein;
a Cas protein derived from *Neisseria meningitidis,* for example, Cas9 protein;
a Cas protein derived from *Pasteurella* sp., e.g., *Pasteurella multocida,* for example, Cas9 protein; and
a Cas protein derived from *Francisella* sp., e.g., *Francisella novicida,* for example, Cas9 protein, but is not limited thereto.

The Cpf1 protein, which is an endonuclease in a new CRISPR system distinguished from the CRISPR/Cas system, is small in size relative to Cas9, requires no tracrRNA, and can act with the guidance of single guide RNA. In addition, the Cpf1 protein recognizes a thymine-rich PAM (protospacer-adjacent motif) sequence and cleaves DNA double strands to form a cohesive end (cohesive double-strand break).

By way of example, the Cpf1 protein may be derived from *Candidatus* sp., *Lachnospira* sp., *Butvrivibrio* sp., *Peregrinibacteria* sp., *Acidominococcus* sp., *Porphyromonas* sp., *Prevotella* sp., *Francisella* sp., *Candidatus Methanoplasma,* or *Eubacterium* sp., e.g., from microbes such as *Parcubacteria* bacterium (GWC2011_GWC2_44_17), *Lachnospiraceae* bacterium (MC2017), *Butyrivibrio proteoclasiicus, Peregrinibacteria* bacterium (GW2011_GWA 33_10), *Acidaminococcus* sp. (BV3L6), *Porphyromonas* macacae, *Lachnospiraceae* bacterium (ND2006), *Porphyromonas crevioricanis, Prevotella disiens, Moraxella bovoculi* (237), *Smiihella* sp. (SC_KO8D17), *Leptospira inadai, Lachnospiraceae* bacterium (MA2020), *Francisella novicida* (U112), *Candidatus Methanoplasma termitum, Candidatus Paceibacter, Eubacterium eligens,* etc., but is not limited thereto.

The target-specific nuclease may be isolated from microbes or may be an artificial or non-naturally occurring enzyme as obtained by recombination or synthesis. For use, the target-specific nuclease may be in the form of an mRNA pre-described or a protein pre-produced *in vitro* or may be included in a recombinant vector so as to be expressed in target cells or *in vivo.* In an embodiment, the target-specific nuclease (e.g., Cas9, Cpf1, etc.) may be a recombinant protein made with a recombinant DNA (rDNA). The term "recombinant DNA" means a DNA molecule formed by artificial methods of genetic recombination (such as molecular cloning) to bring together homologous or heterologous genetic materials from multiple sources. For use in producing a target-specific nuclease by expression in a suitable organism *(in vivo* or *in vitro),* recombinant DNA may have a nucleotide sequence that is reconstituted with optimal codons for expression in the organism which are selected from codons coding for a protein to be produced.

The target-specific nuclease used herein may be a mutant target-specific nuclease in an altered form. The mutant target-specific nuclease may refer to a target-specific nuclease lacking endonuclease activity of cleaving double strand DNA and may be, for example, at least one selected from among mutant target-specific nucleases mutated to lack endonuclease activity but to retain nickase activity and mutant target-specific nucleases mutated to lack both endonuclease and nickase activities. When the mutant target-specific nuclease has nickase activity, a nick may be introduced to a strand on which base conversion (e.g., conversion of cytidine to uridine) is performed by the deaminase or an opposite strand (e.g., a strand paired to the strand on which base conversion happens) (for example, a nick is introduced between nucleotides at positions 3 and 4 in the 5'-end direction on the PAM sequence) simultaneously with or successively to the base conversion irrespective of the order. As such, the mutation of the target-specific nuclease (e.g., amino acid substitution, etc.) may occur at least in the catalytically active domain of the nuclease (for example, RuvC catalyst domain for Cas9). In an embodiment, when the target-specific nuclease is a *Streptococcus* pyogenes-derived Cas9 protein (SwissProt Accession number Q99ZW2(NP_269215.1); SEQ ID NO: 4), the mutation may be amino acid substitution at least one position selected from the group consisting of a catalytic aspartate residue (e.g., aspartic acid at position 10 (D10) for SEQ ID NO: 4, etc.), glutamic acid at position 762 (E762), histidine at position 840 (H840), asparagine at position 854 (N854), asparagine at position 863 (N863), and aspartic acid at position 986 (D986) on the sequence of SEQ ID NO: 4. A different amino acid to be substituted for the amino acid residues may be alanine, but is not limited thereto.

In another embodiment, the mutant target-specific nuclease may be a mutant that recognizes a PAM sequence different from that recognized by wild-type Cas9 protein. For example, the mutant target-specific nuclease may be a mutant in which at least one, for example, all of the three amino acid residues of aspartic acid at position 1135 (D1135), arginine at position 1335 (R1335), and threonine at position 1337 (T1337) of the *Streptococcus* pyogenes-derived Cas9 protein are substituted with different amino acids to recognize NGA (N is any residue selected from among A, T, G, and C) different from the PAM sequence (NGG) of wild-type Cas9.

In one embodiment, the mutant target-specific nuclease may have the amino acid sequence (SEQ ID NO: 4) of *Streptococcus* pyogenes-derived Cas9 protein on which amino acid substitution has been made for:
(1) D10, H840, or D10 + H840;
(2) D1135, R1335, T1337, or D1135 + R1335 + T1337; or
(3) both of (1) and (2) residues.

As used herein, the term "(a) different amino acids" means (an) amino acids selected from among alanine, isoleucine, leucine, methionine, phenylalanine, proline, tryptophan, valine, asparagine, cysteine, glutamine, glycine, serine, threonine, tyrosine, aspartic acid, glutamic acid, arginine, histidine, lysine, and all variants thereof, exclusive of the amino acid retained at the original mutation positions in wild-type proteins. In one embodiment, the "(a) different amino acids" may be alanine, valine, glutamine, or arginine.

In one embodiment, the mutant target-specific nuclease may be a modified Cas9 protein that lacks endonuclease activity (e.g., but retaining nickase activity or lacking both endonuclease activity and nickase activity) or which recognizes a PAM sequence different from that recognized by wild-type Cas9. For example, the modified Cas9 protein may be a mutant of the *Streptococcus pyogenes-derived* Cas9 protein (SEQ ID NO: 4), wherein
(1) mutation (e.g., substitution with a different amino acid) is introduced to D10 or H840 to lack endonuclease activity but retain nickase activity or to both D10 and H840 to lack both endonuclease activity and nickase activity;
(2) mutation (e.g., substitution with a different amino acid) is introduced to at least one or all of D1135, R1335, and T1337 to recognize a PAM sequence different from the wild type; or
(3) both the mutations of (1) and (2) are introduced to retain nickase activity and recognize a PAM sequence different from the wild type or to lack both endonuclease activity and nickase activity and recognize a PAM sequence different from the wild type.

By way of example, a mutation at D10 in the Cas9 protein may be D10A mutation (means substitution of A for D at position 10 in Cas9 protein; hereinafter, mutations introduced to Cas9 are expressed in the same manner), a mutation at H840 may be H840A, and mutations at D1135, R1335, and T1337 may be D1135V, R1335Q, and T1337R, respectively.

Unless otherwise stated herein, the term "nuclease" refers to "target-specific nuclease", such as Cas9, Cpf1, etc., as described above.

The nuclease may be isolated from microbes or may be an artificial or non-naturally occurring enzyme as obtained by recombination or synthesis. In an embodiment, the nuclease (e.g., Cas9, Cpf1, etc.) may be a recombinant protein made with a recombinant DNA (rDNA). The term "recombinant DNA" means a DNA molecule formed by artificial methods of genetic recombination, such as molecular cloning, to bring together homologous or heterologous genetic materials from multiple organism sources. For use in producing a target-specific nuclease by expression in a suitable organism (*in vivo* or *in vitro*)*,* for example, recombinant DNA may have a nucleotide sequence that is reconstituted with optimal codons for expression in the organism which are selected from codons coding for a protein to be produced.

The nuclease may be used in the form of a protein, a nucleic acid molecule coding therefor (e.g., DNA or mRNA), a ribonucleoprotein in which the protein is associated with a guide RNA, a nucleic acid molecule coding for the ribonucleoprotein, or a recombinant vector carrying the nucleic acid molecule.

The deaminase and the nuclease, and/or nucleic acid molecules coding therefor may be in the form that can be translocated into, act within, and/or be expressed within the nucleus.

The deaminase and the nuclease may take a form that is easy to introduce to cells. For example, the deaminase and the nuclease may be linked to a cell penetrating peptide and/or a protein transduction domain. The protein transduction domain may be poly-arginine or an HIV-derived TAT protein, but is not limited thereto.

Because there are various kinds of the cell penetrating peptide or the protein transduction domain in addition to the stated examples, a person skilled in the art may make application of various kinds without limitations to the examples.

In addition, the deaminase and the nuclease, and/or nucleic acid molecules coding therefor may further comprise a nuclear localization signal (NLS) sequence or a nucleic acid sequence coding therefor. Therefore, an expression cassette including a deaminase-encoding nucleic acid molecule and/or a nuclease-encoding nucleic acid molecule may further comprise a regulatory sequence such as a promoter sequence for expressing the deaminase and/or nuclease and optionally an NLS sequence (SEQ ID NO: 13). The NLS sequence is well known in the art.

The deaminase and the nuclease, and/or the nucleic acid coding therefor may be linked to a tag for isolation and/or purification or a nucleic acid coding for the tag. For example, the tag may be selected from the group consisting of small peptide tags, such as His tag, Flag tag, S tag, etc., GST (Glutathione S-transferase) tag, and MBP (Maltose binding protein) tag, but is not limited thereto.

In addition, the base editing composition used in the present disclosure may further comprise a uracil DNA glycosylase inhibitor (UGI) or a coding gene therefor (in the form of a recombinant vector carrying the coding DNA or in the form of mRNA transcribed *in vitro*)*.* The presence of a uracil DNA glycosylase inhibitor in the base editing composition allows for an increased ratio of the conversion of a specific base by deaminase (i.e., conversion from C to T by cytosine deaminase), compared to the absence thereof. On the other hand, when not further including a uracil DNA glycosylase inhibitor, the base editing composition increases a ratio of substitutions for bases other than a specific base (e.g., substitution of C for T by cytosine deaminase) (that is, substitutions are made on various bases). In one embodiment, the uracil DNA glycosylase inhibitor may be encoded by SEQ ID NO: 12, but is not limited thereto.

As used herein, the term "guide RNA" refers to an RNA that includes a targeting sequence hybridizable with a specific base sequence (target sequence) of a target site in a target gene and functions to associate with a nuclease, such as Cas proteins, Cpf1, etc., and guide the nuclease to a target gene (or target site) *in vitro* or *in vivo* (or cells).

The guide RNA may be suitably selected depending on kinds of the nuclease to be complexed therewith and/or origin microorganisms thereof.

For example, the guide RNA may be at least one selected from the group consisting of:
CRISPR RNA (crRNA) including a region (targeting sequence) hybridizable with a target sequence;
trans-activating crRNA (tracrRNA) including a region interacting with a nuclease such as Cas protein, Cpf1, etc.; and
single guide RNA (sgRNA) in which main regions of crRNA and tracrRNA (e.g., a crRNA region including a targeting sequence or a tracrRNA region interacting with nuclease) are fused to each other.

In detail, the guide RNA may be a dual RNA including CRISPR RNA (crRNA) and trans-activating crRNA (tracrRNA) or a single guide RNA (sgRNA) including main regions of crRNA and tracrRNA.

The sgRNA may include a region (named "spacer region", "target DNA recognition sequence", "base pairing region", etc.) having a complementary sequence (targeting sequence) to a target sequence in a target gene (target site), and a hairpin structure for binding to a Cas protein. In greater detail, the sgRNA may include a region having a complementary sequence (targeting sequence) to a target sequence in a target gene, a hairpin structure for binding to a Cas protein, and a terminator sequence. These moieties may exist sequentially in the direction from 5' to 3', but is not limited thereto. So long as it includes main regions of crRNA and tracrRNA and a complementary sequence to a target DNA, any guide RNA can be used in the present disclosure.

For editing a target gene, for example, the Cas9 protein requires two guide RNAs, that is, a CRISPR RNA (crRNA) having a nucleotide sequence hybridizable with a target site in the target gene and a trans-activating crRNA (tracrRNA) interacting with the Cas9 protein. In this context, the crRNA and the tracrRNA may be coupled to each other to form a crRNA:tracrRNA duplex or connected to each other via a linker so that the RNAs can be used in the form of a single guide RNA (sgRNA). In one embodiment, when a *Streptococcus* pyogenes-derived Cas9 protein is used, the sgRNA may form a hairpin structure (stem-loop structure) in which the entirety or a part of the crRNA having a hybridizable nucleotide sequence is connected to the entirety or a part of the tracrRNA including an interacting region with the Cas9 protein via a linker (responsible for the loop structure).

The guide RNA, specially, crRNA or sgRNA, includes a targeting sequence complementary to a target sequence in a target gene and may contain one or more, for example, 1-10, 1-5, or 1-3 additional nucleotides at an upstream region of crRNA or sgRNA, particularly at the 5' end of sgRNA or the 5' end of crRNA of dual RNA. The additional nucleotide(s) may be guanine(s) (G), but are not limited thereto.

In another embodiment, when the nuclease is Cpf1, the guide RNA may include crRNA and may be appropriately selected, depending on kinds of the Cpf1 protein to be complexed therewith and/or origin microorganisms thereof.

Concrete sequences of the guide RNA may be appropriately selected depending on kinds of the nuclease (Cas9 or Cpf1) (i.e., origin microorganisms thereof) and are an optional matter which could easily be understood by a person skilled in the art.

When a *Streptococcus* pyogenes-derived Cas9 protein is used as a target-specific nuclease, crRNA may be represented by the following General Formula 1:

5'-(N_{cas9})ₗ-(GUUUUAGAGCUA)-(X_{cas9})ₘ-3' (General Formula 1)

Wherein:
N_{cas9} is a targeting sequence, that is, a region determined according to a sequence at a target site in a target gene (i.e., a sequence hybridizable with a sequence of a target site), I represents a number of nucleotides included in the targeting sequence and may be an integer of 15 to 30, 17 to 23 or 18 to 22, for example, 20;
the region including 12 consecutive nucleotides (GUUUUAGAGCUA; SEQ ID NO: 1) adjacent to the 3'-end of the targeting sequence is essential for crRNA;
X_{cas9} is a region including m nucleotides present at the 3'-terminal site of crRNA (that is, present adjacent to the 3'-end of the essential region); and
m may be an integer of 8 to 12, for example, 11 wherein the m nucleotides may be the same or different and are independently selected from the group consisting of A, U, C, and G.

In an embodiment, the X_{cas9} may include, but is not limited to, UGCUGUUUUG (SEQ ID NO: 2).

In addition, the tracrRNA may be represented by the following General Formula 2: 
5'-(Y_{cas9})ₚ-
(UAGCAAGUUAAAAUAAGGCUAGUCCGUUAUCAACUUGAAAAAGUGGCACC
GAGUCGGUGC)-3' (General Formula 2)
 wherein,
the region represented by 60 nucleotides (UAGCAAGUUAAAAUAAGGCUAGUCCGUUAUCAACUUGAAAAAGUGGCACC GAGUCGGUGC) (SEQ ID NO: 3) is essential for tracrRNA,
Ycas9 is a region including p nucleotides present adjacent to the 3'-end of the essential region, and
p is an integer of 6 to 20, for example, 8 to 19 wherein the p nucleotides may be the same or different and are independently selected from the group consisting of A, U, C, and G.

Further, sgRNA may form a hairpin structure (stem-loop structure) in which a crRNA moiety including the targeting sequence and the essential region thereof and a tracrRNA moiety including the essential region (60 nucleotides) thereof are connected to each other via an oligonucleotide linker (responsible for the loop structure). In greater detail, the sgRNA may have a hairpin structure in which a crRNA moiety including the targeting sequence and an essential region thereof is coupled with the tracrRNA moiety including the essential region thereof to form a double-strand RNA molecule with connection between the 3' end of the crRNA moiety and the 5' end of the tracrRNA moiety via an oligonucleotide linker.

In one embodiment, the sgRNA may be represented by the following General Formula 3: 
5'-(N_{cas9})ₗ-(GUUUUAGAGCUA)-(oligonucleotide linker)-
(UAGCAAGUUAAAAUAAGGCUAGUCCGUUAUCAACUUGAAAAAGUGGCACC
GAGUCGGUGC)-3' (General Formula 3)
 wherein (N_{cas9})ₗ is a targeting sequence defined as in General Formula 1.

The oligonucleotide linker included in the sgRNA may be 3-5 nucleotides long, for example 4 nucleotides long in which the nucleotides may be the same or different and are independently selected from the group consisting of A, U, C, and G.

The crRNA or sgRNA may further contain 1 to 3 guanines (G) at the 5' end thereof (that is, the 5' end of the targeting sequence of crRNA).

The tracrRNA or sgRNA may further comprise a terminator inclusive of 5 to 7 uracil (U) residues at the 3' end of the essential region (60 nt long) of tracrRNA.

The target sequence for the guide RNA may be about 17 to about 23 or about 18 to about 22, for example, 20 consecutive nucleotides adjacent to the 5' end of PAM (Protospacer Adjacent Motif (for S. pyogenes Cas9, 5'-NGG-3' (N is A, T, G, or C)) on a target DNA.

As used herein, the term "the targeting sequence" of guide RNA hybridizable with the target sequence for the guide RNA refers to a nucleotide sequence having a sequence complementarity of 50 % or higher, 60 % or higher, 70 % or higher, 80 % or higher, 90 % or higher, 95 % or higher, 99 % or higher, or 100 % to a nucleotide sequence of a complementary strand to a DNA strand on which the target sequence exists (i.e., a DNA strand having a PAM sequence (5'-NGG-3' (N is A, T, G, or C))) and thus can complimentarily couple with a nucleotide sequence of the complementary strand.

In the description, a nucleic acid sequence at a target site is represented by that of the strand on which a PAM sequence exists among two DNA strands in a region of a target gene. In this regard, the DNA strand to which the guide RNA couples is complementary to a strand on which a PAM sequence exists. Hence, the targeting sequence included in the guide RNA has the same nucleic acid sequence as a sequence at an on-target site, with the exception that U is employed instead of T due to the RNA property. In other words, a targeting sequence of the guide RNA and a target sequence are represented by the same nucleic acid sequence with the exception that T and U are interchanged, in the description.

The guide RNA may be used in the form of RNA (or may be contained in the composition) or in the form of a plasmid carrying a DNA coding for the RNA (or may be contained in the composition).

### Advantageous Effect

As described in the specification, base editing (for example, single nucleotide substitution) can be performed by introducing a deaminase and a target-specific nuclease in the form of, for example, mRNA and RNP to mammalian (e.g., murine) cells through microinjection or electroporation. When such base editing is conducted in non-human mammalian embryos, the embryos can be successfully developed to pups having point mutations induced by the base editing. Taken together, the results indicate that the deaminase and the target-specific nuclease can be used to construct various animal models in which single amino acid substitutions and nonsense mutations are induced

### Brief Description of the Drawings

FIGS. 1a to 1e pertain to dystrophin-deficient mutant mice generated by cytidine deaminase-mediated base editing,
FIG. 1a shows the nucleotide sequence at the target site in the dystrophin locus (Dmd) (the PAM sequence and the sgRNA target sequence are shown in blue and black, respectively and the nucleotide substituted by cytidine deaminase-mediated base editing is shown in red),

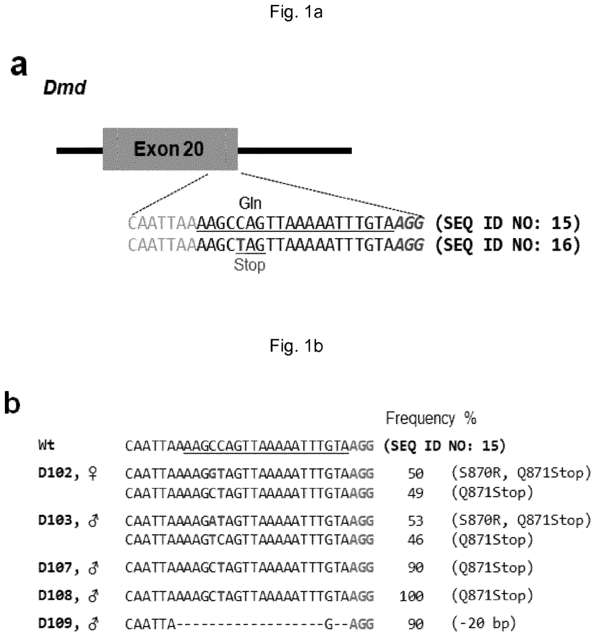

FIG. 1b shows alignments of nucleotide sequences at the target site in the Dmd gene from newborn pups that developed after base editor 3 (BE3) (rAPOBEC1-nCas9-UGI)-encoding mRNA and sgRNA hybridizable with the target sequence of the Dmd gene of FIG. 1a were microinjected into mouse zygotes (Wt, wild-type; the target sequence is underlined; the PAM sequence and substituted nucleotides are shown in blue and red, respectively; the column on the right indicates frequencies (%) of mutant (base-substituted) alleles and '-' stands for the absence of nucleotides at corresponding positions (deletion); Numerals on the left indicate Dmd mutant mouse numbers),
FIG. 1c shows Sanger sequencing chromatograms of DNA from wild-type and Dmd mutant mice D108 (the arrow indicates the substituted nucleotide, showing the conversion of a Gln codon to a stop codon by base substitution),
FIG. 1d shows histological analysis (fluorescence analysis) results of tibialis anterior (TA) muscles from wild-type and Dmd mutant mice D108 (Laminin: control; muscles were dissected from 4-week-old wild-type or Dmd mutant mice D108 and frozen in liquid nitrogen-cooled isopentane; scale bars: 50 µm).
FIG. 1e summarizes procedures of inducing base substitutions at the target site in the Dmd gene and the results.
FIG. 2a to 2e pertain to the generation of albinism mice by cytidine deaminase-mediated base editing,

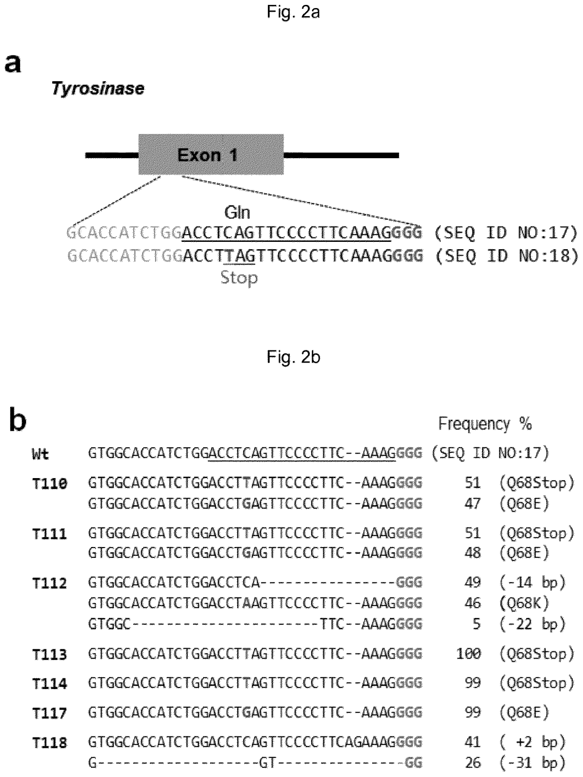

FIG. 2a shows the nucleotide sequence at the target site in the tyrosinase gene (Tyr) (the PAM sequence (NGG) and the sgRNA target sequence are shown in blue and black, respectively; the nucleotides substituted by cytidine deaminase-mediated base editing is shown in red),
FIG. 2b shows alignments of nucleotide sequences at the target site in the Tyr gene from newborn pups that developed after base editor 3 (BE3) (rAPOBEC1-nCas9-UGI) RNP (complex of BE3 and sgRNA hybridizable with the target sequence of the Tyr gene of FIG. 2a) was introduced to mouse zygotes by electroporation (Wt, wild-type; the target sequence is underlined; the PAM sequence and substitutions are shown in blue and red, respectively; the column on the right indicates frequencies (%) of mutant (base-substituted) alleles and '-' stands for the absence of nucleotides at corresponding positions (deletion); Numerals on the left indicate Tyr mutant mouse numbers),

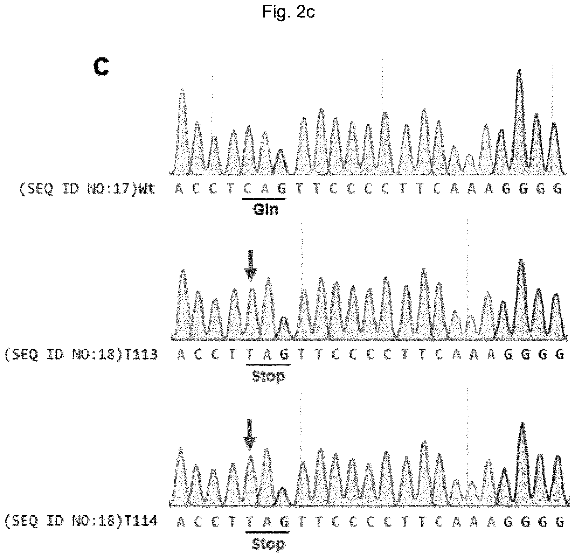

FIG. 2c shows Sanger sequencing chromatograms of DNA from wild-type and Tyr mutant mice T113 and T114 (the arrows indicate the substituted nucleotides, showing the conversion of a Gln codon to a stop codon by base substitution),

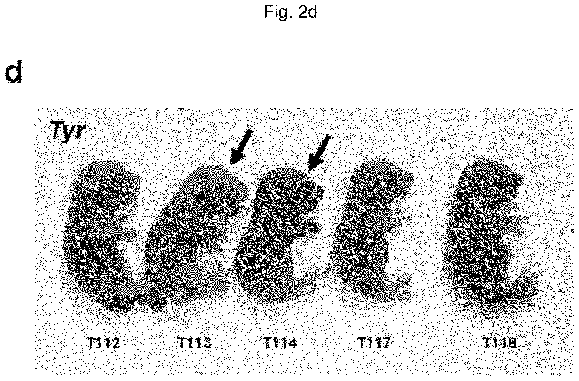

FIG. 2d shows an albino phenotype in the eyes of Tyr mutant newborn pups that developed after electroporation of the BE3 RNP (T113 and T114 indicated by arrows),

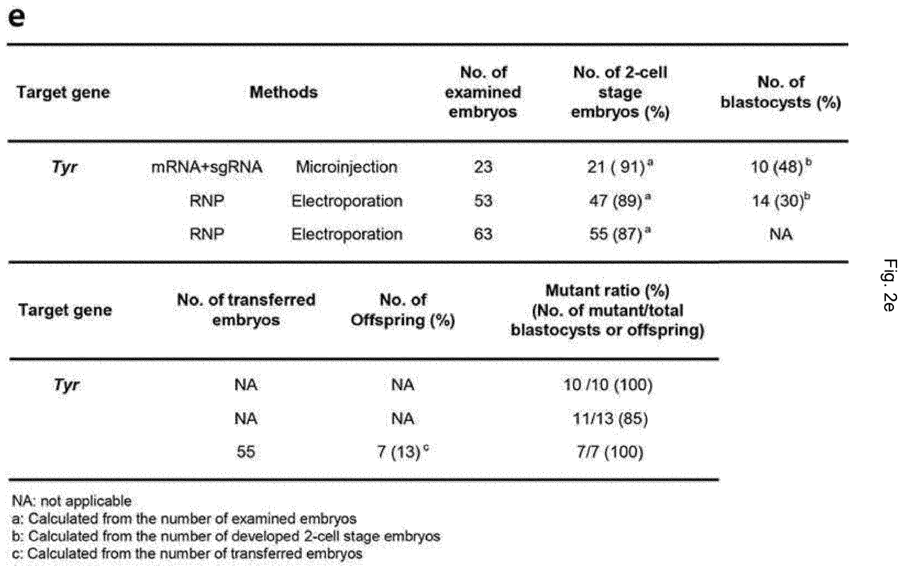

FIG. 2e summarizes procedures of inducing base substitutions at the target site in the Tyr gene and the results.

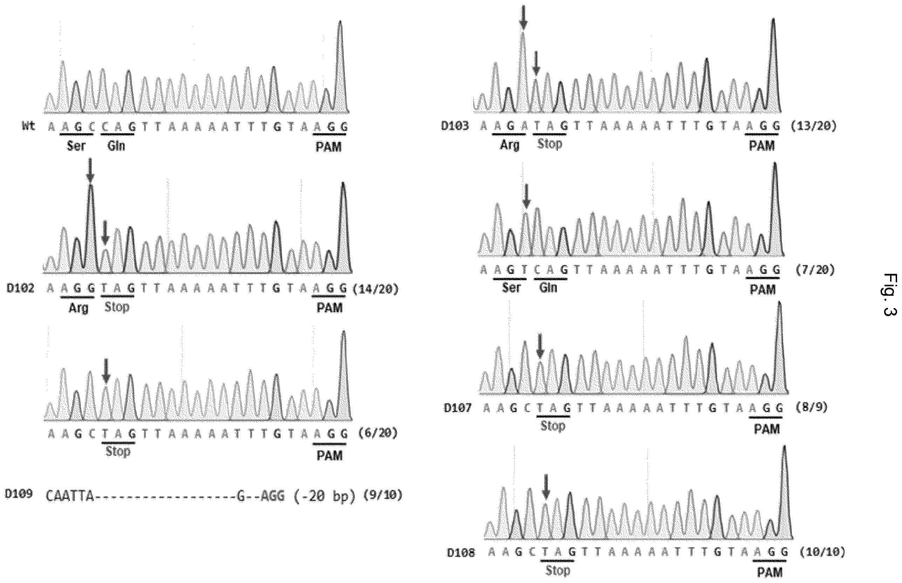

FIGS. 3a and 3b are alignments of the nucleotide sequences at the target sites in the target genes (Dmd and Tyr) of blastocysts that developed after microinjection of BE3-encoding mRNA and sgRNA into mouse zygotes, showing the induction of targeted mutations in mouse embryos by microinjection of BE3(deaminase-Cas9) mRNA and sgRNA thereinto (3a: Dmd mutation result; 3b: Tyr mutation result; Wt, wild-type; the target sequences are underlined; the PAM sequences and substitutions are shown in blue and red, respectively; the column on the right indicates frequencies (%) of mutant (base-substituted) alleles and '-' stands for the absence of nucleotides at corresponding positions (deletion); Numerals on the left indicate Tyr mutant mouse numbers),
FIG. 3 shows Sanger sequencing chromatograms of DNA from wild-type and Dmd mutant mice (the arrows indicate substituted nucleotides).

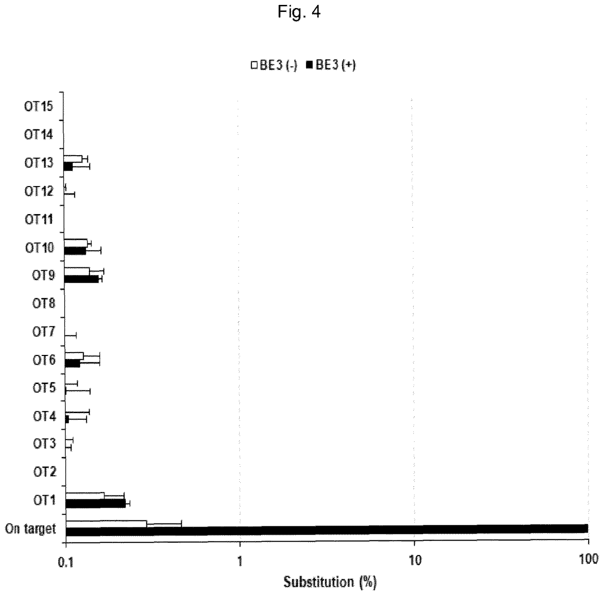

FIG. 4 is a graph of results after testing whether or not Dmd mutations are generated at potential off-target sites, showing that no off-target mutations are detectably induced at the off-target sites (Targeted deep sequencing was used to measure mutant ratios (base editing efficiencies) (%) at potential off-target sites in Dmd mutant mice (n = 3); mismatched nucleotides and PAM sequences are shown in red and blue, respectively).

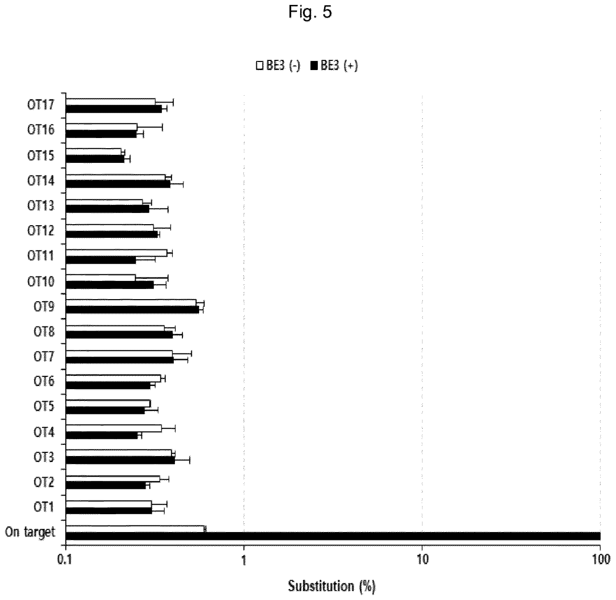

FIG. 5 is a graph of results after testing whether or not Tyr mutations are generated at potential off-target sites, showing that no off-target mutations are detectably induced at the off-target sites (Targeted deep sequencing was used to measure mutant ratios (base editing efficiencies) (%) at potential off-target sites in Tyr mutant mice (n = 2); mismatched nucleotides and PAM sequences are shown in red and blue, respectively).

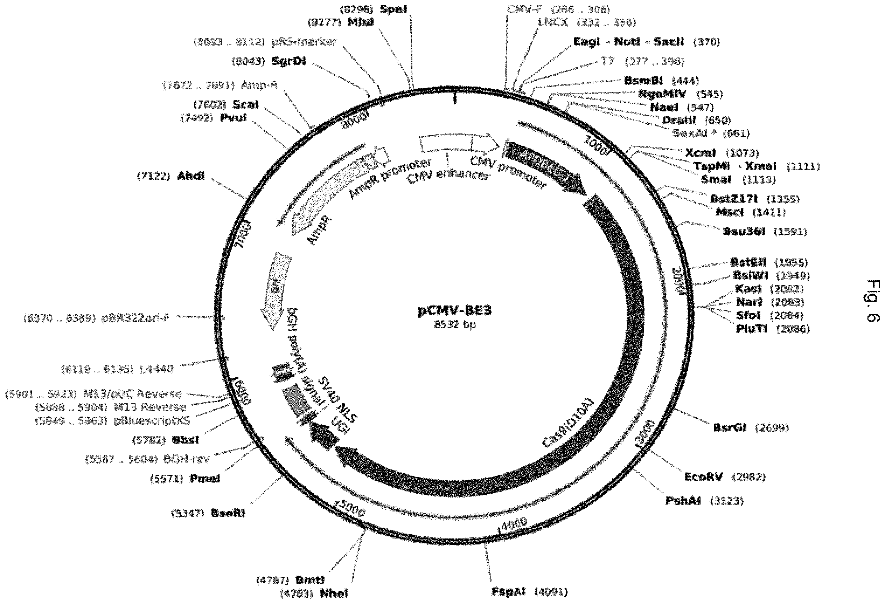

FIG. 6 is a cleavage map of the pCMV-BE3 vector.

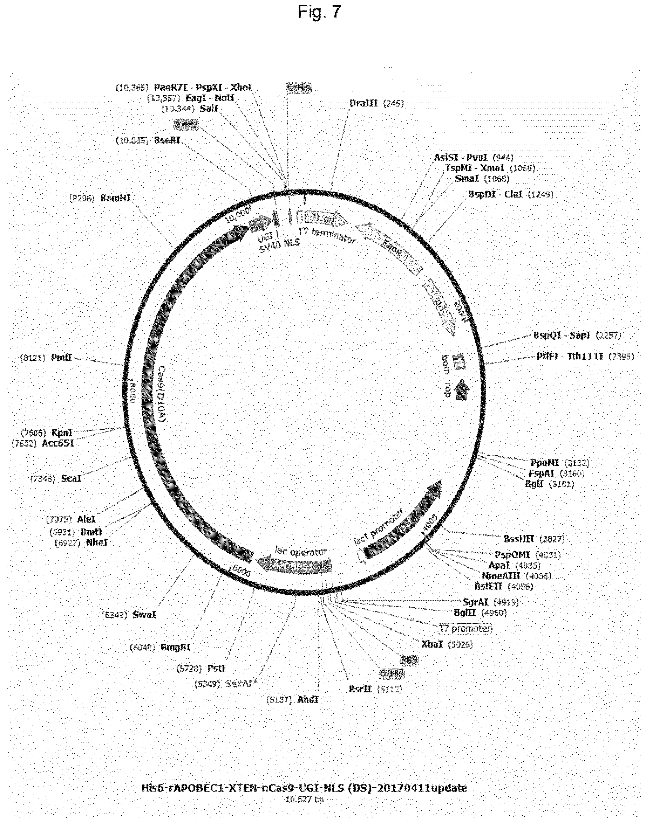

FIG. 7 is a cleavage map of the pET-Hisx6-rAPOBEC1-XTEN-nCas9-UGI-NLS vector.

### Mode for Carrying Out the Invention

Hereafter, the present disclosure will be described in detail by examples. The following examples are intended merely to illustrate the invention and are not construed to restrict the invention.

### EXAMPLE 1: Preparation of BE3 mRNA

After being isolated by digestion from pCMV-BE3 (Addgene; cat. #73021; FIG. 6), rAPOBEC1-XTEN (linker) and UGI (uracil DNA glycosylase inhibitor) were inserted into the pET-nCas9 (D10A)-NLS vector (see Cho, S.W. et al. Analysis of off-target effects of CRISPR/Cas-derived RNA-guided endonucleases and nickases. Genome Res 24, 132-141 (2014)) to construct pET-Hisx6-rAPOBEC1-XTEN-nCas9-UGI-NLS (SEQ ID NO: 7; FIG. 7) which was then used as a BE3 mRNA template.

Sequences of individual regions in pET-Hisx6-rAPOBEC1-XTEN-nCas9-UGI-NLS (SEQ ID NO: 7) are summarized as follows:
His x6: SEQ ID NO: 8;
rAPOBEC1: SEQ ID NO: 9;
XTEN (linker): SEQ ID NO: 10;
nCas9 (D10A): SEQ ID NO: 11;
Linker: TCTGGTGGTTCT (SEQ ID NO: 14)
UGI: SEQ ID NO: 12;
Linker: TCTGGTGGTTCT (SEQ ID NO: 14)
NLS: SEQ ID NO: 13.

PCR was performed on the pET-Hisx6-rAPOBEC1-XTEN-nCas9-UGI-NLS vector with the aid of Phusion High-Fidelity DNA Polymerase (Thermo Scientific) in the presence of primers (F: 5'-GGT GAT GTC GGC GAT ATA GG-3', R: 5'-CCC CAA GGG GTT ATG CTA GT-3') to prepare the mRNA temperature. From the prepared mRNA template, BE3 mRNA was synthesized using an *in vitro* RNA transcription kit (mMESSAGE mMACHINE T7 Ultra kit, Ambion), followed by purification with MEGAclear kit (Ambion).

### EXAMPLE 2: Preparation of sgRNA

A dystrophin gene Dmd and a tyrosinase gene Tyr targeted guide RNA (sgRNA) having the following nucleotide sequence were synthesized and used in subsequent experiments: 
5'-(target sequence)-(GUUUUAGAGCUA; SEQ ID NO: 1)-(nucleotide
linker)-
(UAGCAAGUUAAAAUAAGGCUAGUCCGUUAUCAACUUGAAAAAGUGGCACC
GAGUCGGUGC; SEQ ID NO: 3)-3'
(the target sequence is the same sequence as the underlined nucleotide sequence in FIG. 1a (Dmd) or FIG. 2a (Tyr), with the exception that "T" is converted to "U", and
the nucleotide linker has the nucleotide sequence of GAAA).

The sgRNA was constructed by *in vitro* transcription using T7 RNA polymerase (see Cho, S.W., Kim, S., Kim, J.M. & Kim, J.S. Targeted genome engineering in human cells with the Cas9 RNA-guided endonuclease. Nat Biotechnol 31, 230-232 (2013)).

### EXAMPLE 3: Preparation of Ribonucleoproteins (RNPs)

Rosetta competent cells (EMD Millipore) were transformed with the pET28-Hisx6-rAPOBEC1-XTEN-nCas9(D10A)-UGI-NLS (BE3) expression vector prepared in Reference Example 1 and then incubated with 0.5 mM isopropyl beta-D-1-thiogalactopyranoside (IPTG) at 18°C for 12 to 14 hours to induce expression. Following protein expression, bacterial cells were harvested by centrifugation and the cell pellet was lysed by sonication in a lysis buffer [50 mM NaH2PO4 (pH 8.0), 300 mM NaCl, 10 mM imidazole, 1% Triton X-100, 1 mM PMSF, 1 mM DTT, and 1 mg/ml lysozyme].

The cell lysate thus obtained was subjected to centrifugation at 5,251xg for 30 min to remove cell debris. The soluble lysate was incubated with Ni-NTA beads (Qiagen) at 4°C for 1 hr. Subsequently, the Ni-NTA beads were washed three times with wash buffer [50 mM NaH2PO4 (pH 8.0), 300 mM NaCl, and 20 mM imidazole], followed by eluting BE3 protein with elution buffer [50 mM Tris-HCl (pH 7.6), 150-500 mM NaCl, 10-25% glycerol, and 0.2 M imidazole]. The purified BE3 protein was dialyzed against storage buffer [20 mM HEPES (pH 7.5), 150 mM KCl, 1 mM DTT, and 10% glycerol] and concentrated using Ultracell 100K cellulose column (Millipore). The purity of the protein was analyzed by SDS-PAGE. SgRNA was prepared by *in vitro* transcription using T7 RNA polymerase as described in Example 3.

### EXAMPLE 4: Preparation of Animal

Experiments on mice were conducted after approval by the Institutional Animal Care and Use Committee (IACUC) at Seoul National University. Mice were maintained in a SPF (specific pathogen-free) condition, with a 12/12 hrs light/dark cycle. C57BL/6J and ICR mice were used as an embryo donor and a surrogate mother.

### EXAMPLE 5: Microinjection and Electroporation into Mouse Zygote

Superovulation, embryo collection, microinjection, and electroporation were performed with reference to "Hur, J.K. et al., Targeted mutagenesis in mice by electroporation of Cpf1 ribonucleoproteins. Nat Biotechnol 34, 807-808 (2016)".

For microinjection, a solution containing a complex of BE3 mRNA (10 ng/ul) and sgRNA (100 ng/ul) was diluted in DEPC-treated injection buffer (0.25 mM EDTA, 10 mM Tris, pH 7.4) (see Sung, Y.H. et al. Highly efficient gene knockout in mice and zebrafish with RNA-guided endonucleases. Genome Res 24, 125-131 (2014)) and then injected into pronuclei of one-cell stage zygote at fertilization with the aid of Nikon ECLIPSE Ti micromanipulator and FemtoJet 4i microinjector (Eppendorf).

For electroporation, a BE3-sgRNA RNP complex was introduced to one-cell mouse embryos by electroporation using a NEPA 21 electroporator (NEPA GENE Co. Ltd.) including a glass chamber filled with 100 µl of opti-MEM (Thermo Fisher Scientific) containing the BE3-sgRNA RNP complex (10 µg/100 µl and 6.5 µg/100 µl, respectively) (see Hur, J.K. et al. Targeted mutagenesis in mice by electroporation of Cpf1 ribonucleoproteins. Nat Biotechnol 34, 807-808 (2016)).

After BE3 RNP or mRNA transfer, the embryos were cultured in microdrops of KSOM+AA (Millipore) at 37°C for 4 days under a humidified condition of 5% CO2. Two-cell stage embryos were transplanted to the oviduct of a 0.5-dpc pseudo pregnant surrogate mother.

The procedure is summarized in the following diagram:

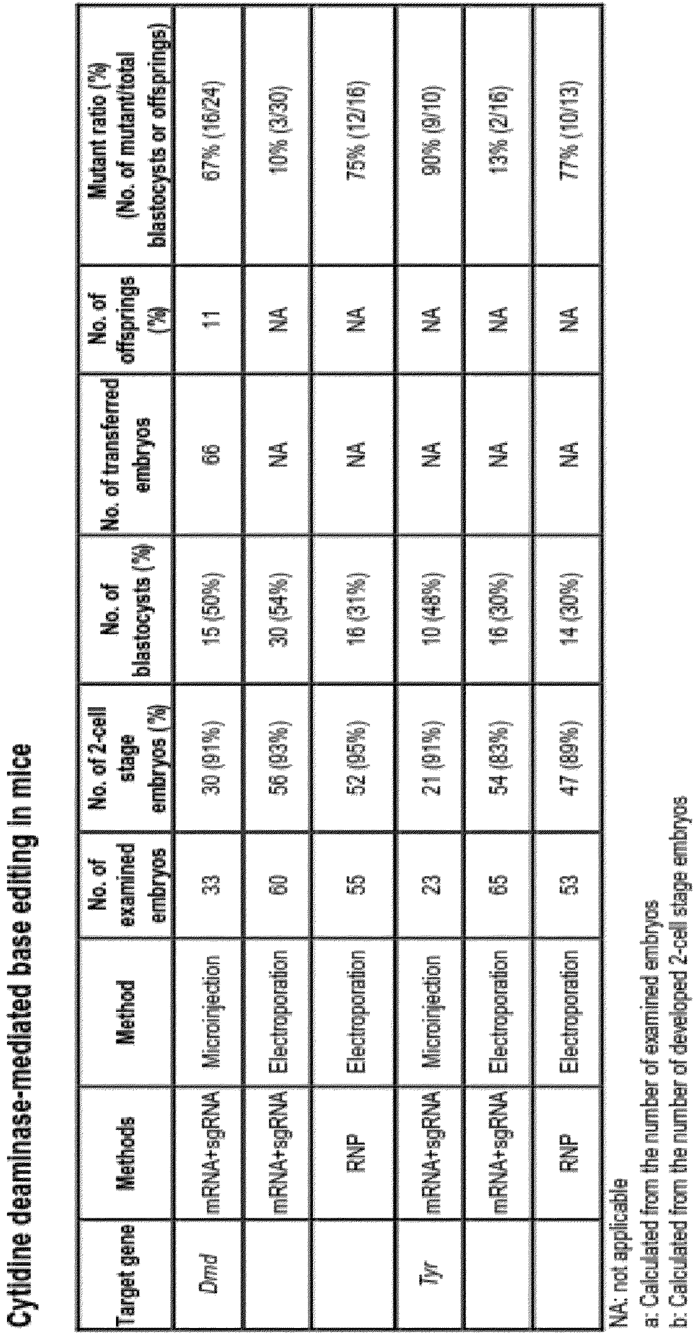

### EXAMPLE 6: Genotyping

For PCR genotyping, genomic DNA was extracted from the blastula stage embryo obtained from the embryo transplanted to the oviduct in Reference Example 4 or from an ear clip of the newborn pups and subjected to targeted deep sequencing and Sanger sequencing.

### EXAMPLE 7: Targeted Deep Sequencing

In this Example, nucleotide sequences were analyzed using targeted deep sequencing as follows.

Target sites or off-target sites were amplified from the genomic DNA extracted in Reference Example 5 with the aid of Phusion polymerase (Thermo Fisher Scientific). Paired-end sequencing of the PCR amplicons was performed using Illumina MiSeq (LAS, Inc. (South Korea) commissioned to perform). Primers used in the amplification of off-target sites are given in Tables 1 and 2, blow.

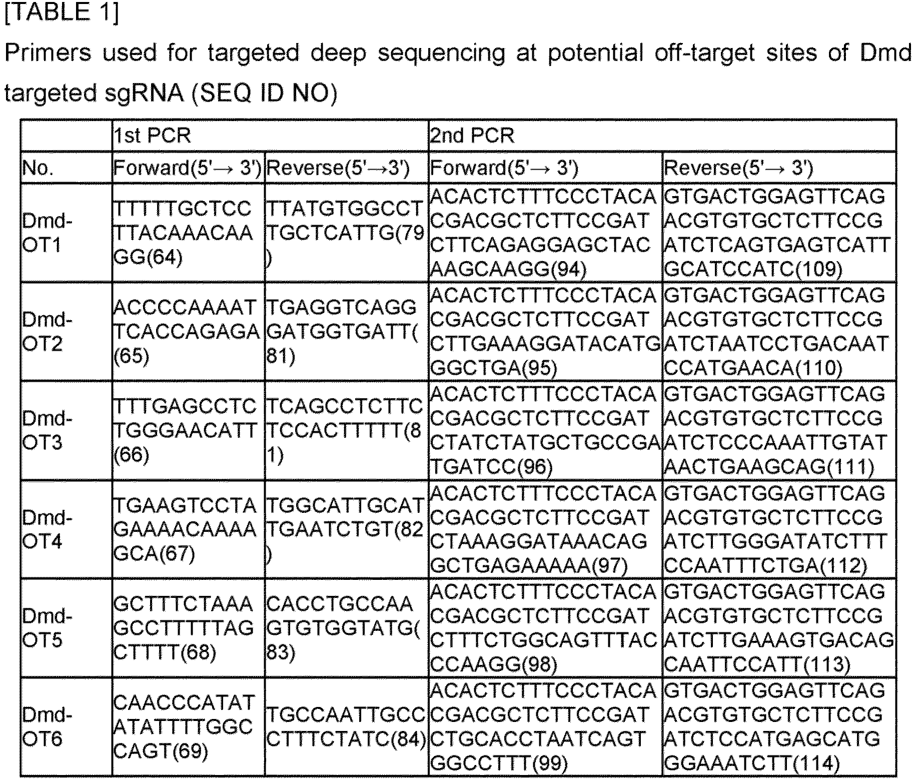

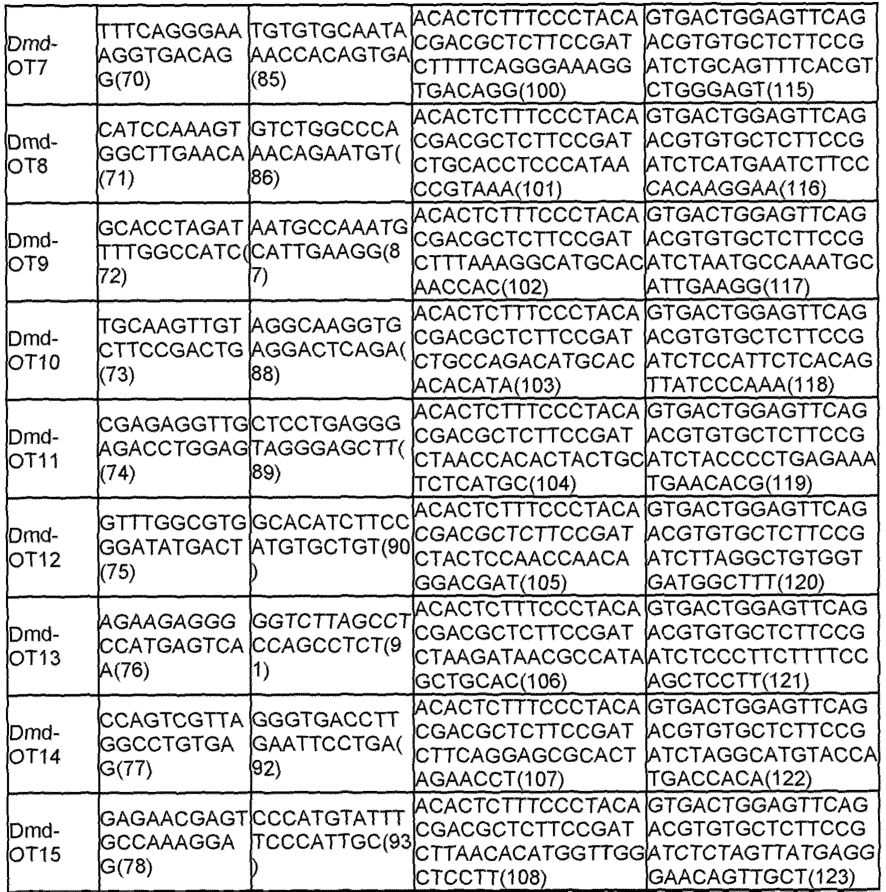

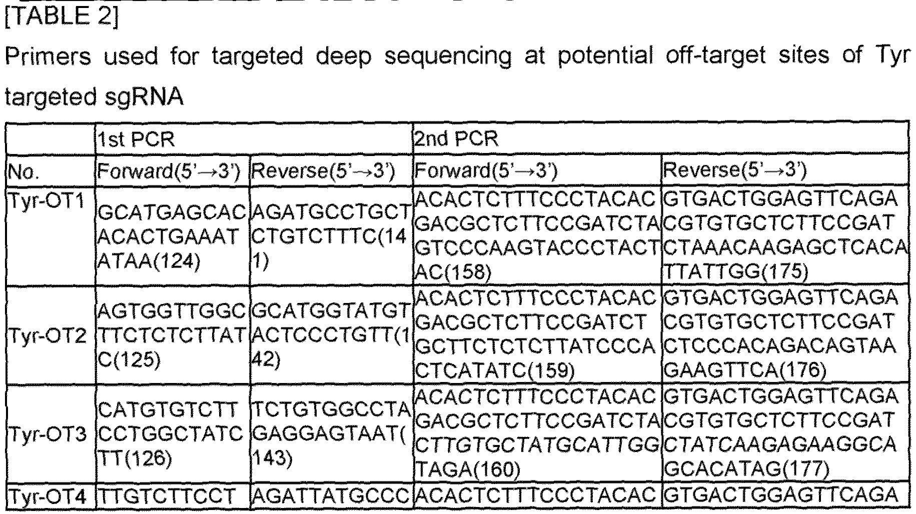

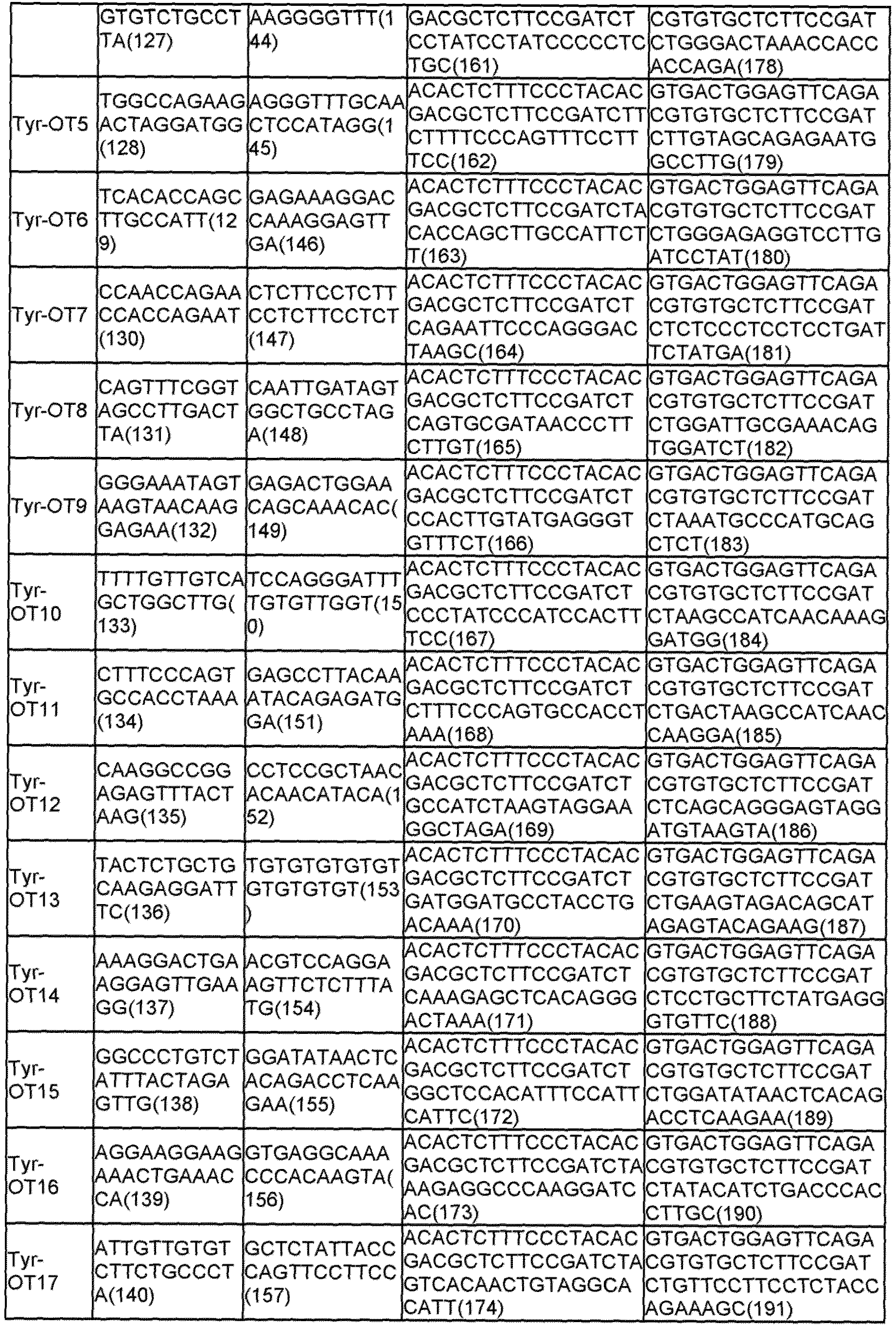

### EXAMPLE 8: Immunofluorescent Staining

Tibialis anterior (TA) muscle sections resected from the mouse were immunostained with a laminin or dystrophin antibody. Laminin was detected using a 1:500 dilution of a rabbit polyclonal antibody (abcam, ab11575) and a 1:1000 dilution of an Alexa Fluor 568 anti-rabbit secondary antibody (Thermo Fisher Scientific) sequentially. For dystrophin detection, a 1:500 dilution of rabbit polyclonal antibody (abcam, ab15277) and a 1:1000 dilution of an Alexa Fluor 488 anti-rabbit secondary antibody (Thermo Fisher Scientific) were used sequentially. The immunofluorescently stained sections were observed with a Leica DMI4000 B fluorescence microscope.

### EXAMPLE 9: Sequencing of Mouse Embryo Having Mutation Induced with BE3

As described in Examples 1-5, Base Editor 3 (BE3) (rAPOBEC1-nCas9-UGI) was introduced to mouse embryonic cells by microinjection to induce a point mutation in each of the dystrophin-encoding gene Dmd and the tyrosinase-encoding gene Tyr.

As shown in FIGS. 1a (Dmd) and 2a (Tyr), the generation of a stop codon would be predicted by single base substitution (C→T) at a target site (underlined sequence sites of upper sequences in FIGS. 1a and 2a) in each gene (in FIGS. 1a and 2a, single base substitutions occurred on lower nucleotide sequences, with substituted (C→T) bases appearing red).

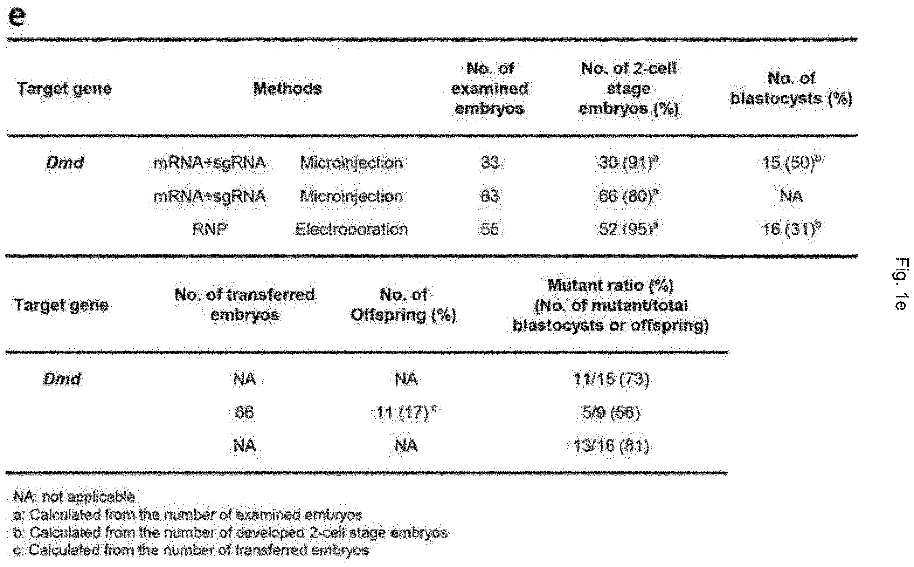

FIGS. 1e and 2e show summaries of procedures of target-specific single base substitution (microinjection or electroporation) and results thereof. As shown in FIGS. 1e and 2e, embryonic mutations were observed at target sites in Dmd and Tyr genes at frequencies of 73 % (11 of 15 for Dmd) and 100 % (10 of 10 for Tyr), respectively.

In addition, nucleotide sequences of target sites in mouse embryos in which mutations had been induced by microinjecting BE3 mRNA and sgRNA were identified by targeted deep sequencing. In greater detail, target-specific mutation was induced by microinjecting mouse BE3 (rAPOBEC1-nCas9-UGI) mRNA and sgRNA into mouse embryos. For this, BE3-encoding mRNA and sgRNA were microinjected to mouse zygotes, and then nucleotide sequences of target sites in the target genes (Dmd and Tyr) in the resulting blastocysts were aligned.

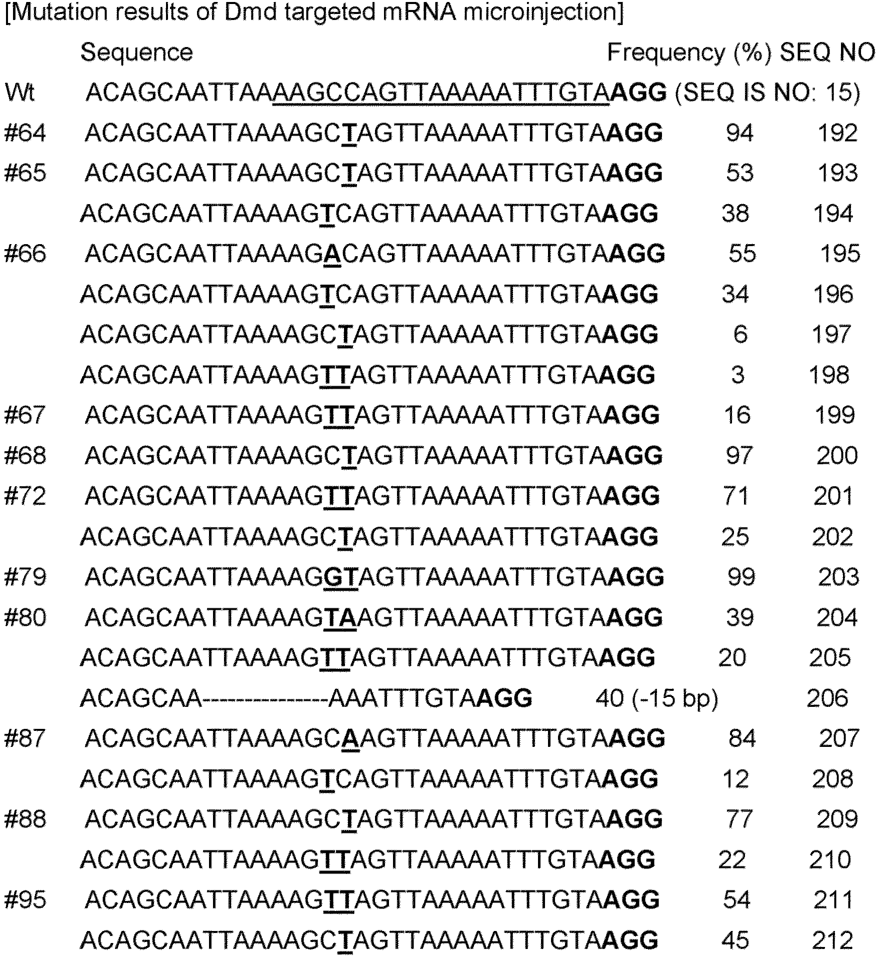

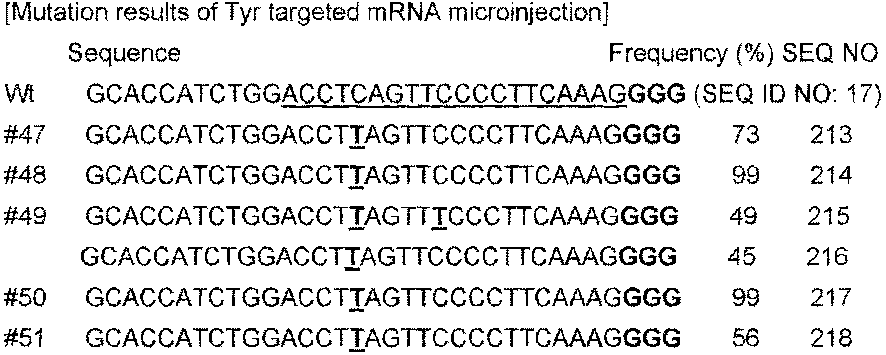

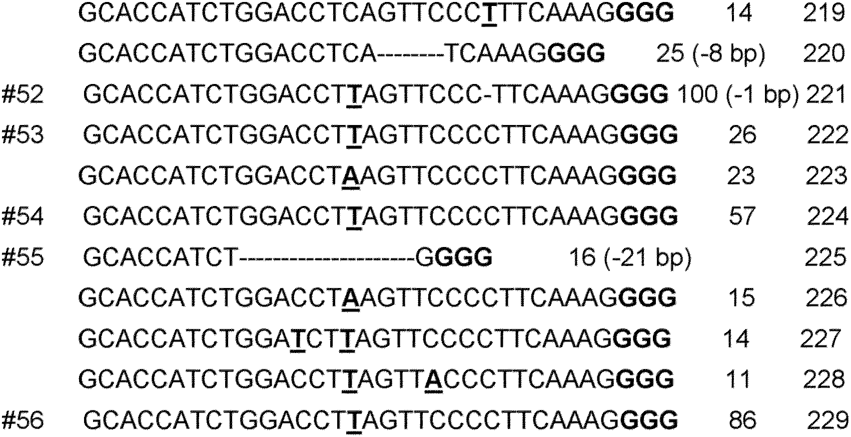

(Wt, wild-type; the target sequence is underlined; the PAM sequence (NGG) is shown in bold; substituted bases are in bold and underlined; the column on the right indicate frequencies (%) of mutant (base substituted) alleles and '-' stands for absence of nucleotides at corresponding positions (deletion); numerals on the left are mutated mouse embryonic cell numbers)

As is understood from the aligned sequences, C→T base substitution is a predominant mutation pattern at both the target sites in the two genes (Dmd and Tyr).

### EXAMPLE 10: Identification and Induction of Mutation in Mouse Subject by Microinjection of BE3 and Dmd Targeted sgRNA

After microinjection of BE3 mRNA and Dmd targeted sgRNA thereinto, mouse embryos were transplanted to the oviduct of foster surrogate mothers (see Example 5) to give mutant newborn pups having point mutation on the Dmd gene thereof (F0).

FIGS. 1b and 3 show analysis results of nucleotide sequences of the target site in the target gene (Dmd) of newborn pups developed after BE3 (rAPOBEC1-nCas9-UGI)-encoding mRNA and sgRNA hybridizable with the nucleotide sequence of the target site in the Dmd gene have been injected into moue zygotes. As can be seen in FIG. 1b, when point mutation was induced in the Dmd gene, five (D102, D103, D107, D108, and D109) among a total of nine mice had mutation at the target site in the Dmd gene. Of the five mutant mice, three subjects (D102, D103, and D108) were found to have one or two mutant allele genes and lack wild-type allelomorphic characteristics. The other two mutant mice (D107 and D109) retained wild-type allele genes in a mosaic pattern at a frequency of 10 %. In addition, as can be seen FIGS. 1b and 3, the mutant mouse D109 exhibited 20-base pair (bp) deletion other than point mutation, demonstrating that the Cas9 nickase included in BE3 retains the activity of inducing indels at the target site.

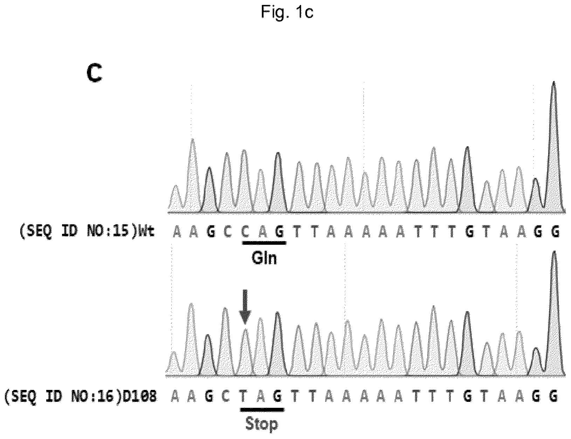

FIG. 1c shows Sanger sequencing chromatograms of the target site in the target gene of wild-type mouse and Dmd mutant mouse D108. As shown in FIG. 1c, the mutant F0 mouse D108, which lacks a wild-type allele gene, had an early stop codon (TAG) introduced by single base substitution (C→T) to the Dmd gene thereof.

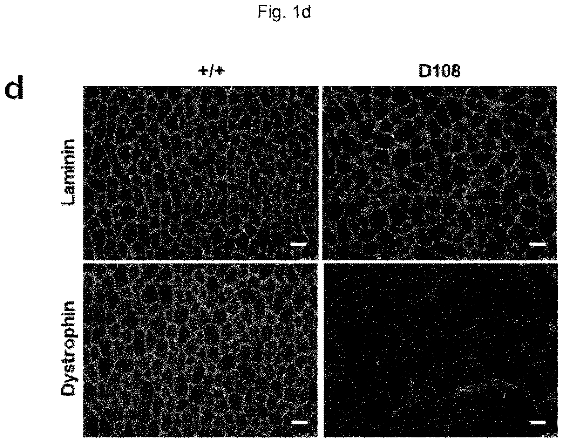

FIG. 1d shows images of immunofluorescent stained TA muscle sections from the wild-type mouse and the Dmd mutant mouse D108 (see Example 8), exhibiting that dystrophin was nearly not expressed in the muscle of the mutant subject (D108). The result implies that the Dmd gene was successfully knocked down by the injection (microinjection of BE3 mRNA and Dmd targeted sgRNA.

### EXAMPLE 11: Identification and Induction of Mutation in Mouse Embryo by Electroporation of RNA Comprising BE3 and sgRNA

BE3 ribonucleproteins (RNPs), prepared in Example 3, including a mixture (rAPOBEC1-nCas9(D10A)-UGI RNP) of the recombinant BE3 protein and the in-vitro transcribed sgRNA were transferred to mouse embryos by electroporation (see Example 5). Four days after electroporation, nucleotide sequences of target sites in the target genes (Dmd and Tyr) of the mouse embryos were analyzed and the results are given as follows:

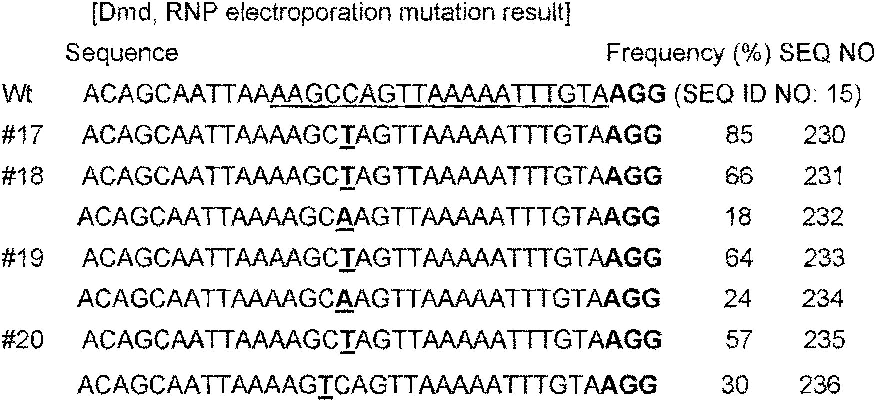

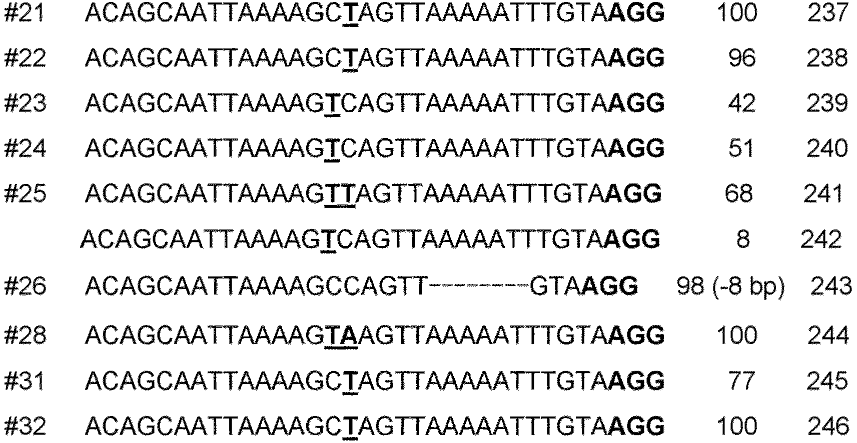

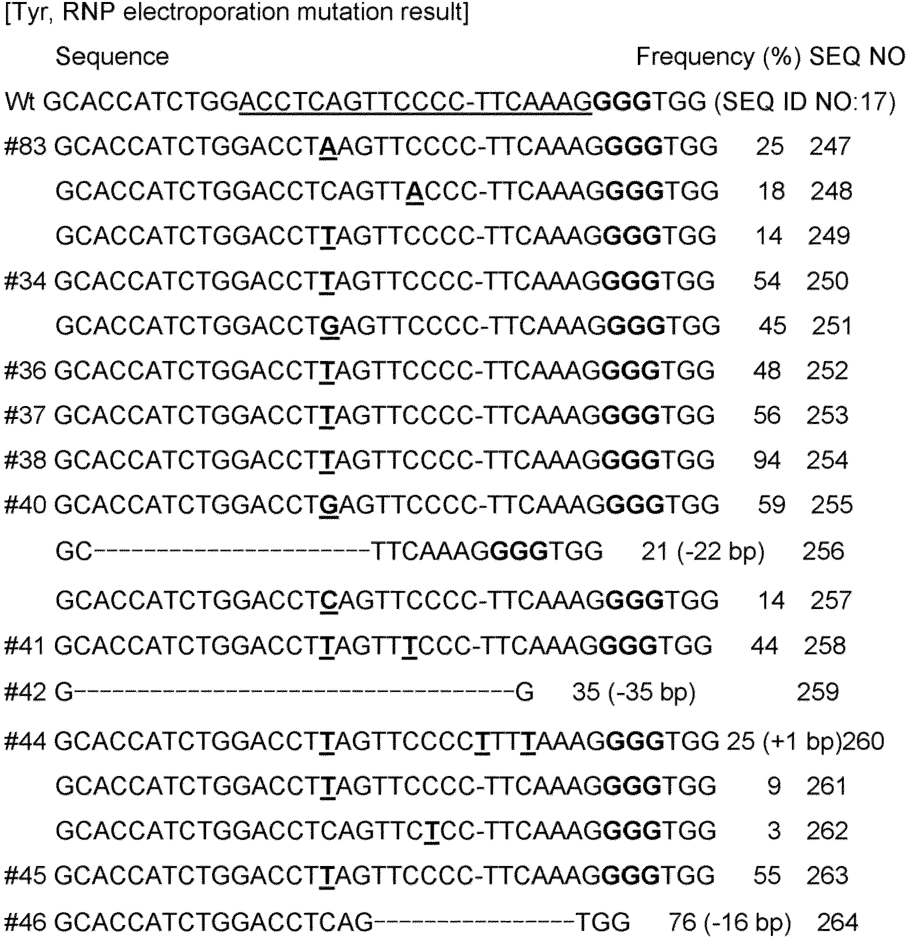

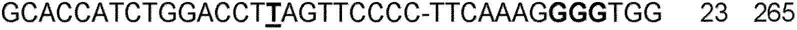

(Wt, wild-type; the target sequence is underlined; the PAM sequence (NGG) is shown in bold; substituted bases are in bold and underlined; the column on the right indicate frequencies (%) of mutant (base substituted) alleles and '-' stands for absence of nucleotides at corresponding positions (deletion); numerals on the left are mutated mouse numbers)

As understood from the results and FIGS. 1e and 2e, the electroporation induced mutations at the Dmd and Tyr target sites in the blastocyst embryos at frequencies of 81 % (13 of 16 for Dmd) and 85 % (11 of 13 for Tyr).

### EXAMPLE 12: Identification and Induction of Mutation in Mouse Subject by Electroporation of BE3 and Tyr Targeted sgRNA

After electroporation of BE3 and Tyr targeted sgRNA thereinto, mouse embryos were transplanted to the oviduct of surrogate mothers (see Example 5) to give mice having point mutation on the Tyr gene thereof (F0).

FIG. 2b shows alignment of nucleotide sequences at the target site in Tyr genes of the mutant newborn pups thus obtained. As shown in FIG. 2b, various mutations were induced at the target site in the Tyr gene of all of the seven mutant newborn pups (T110, T111, T112, T113, T114, T117, and T118).

FIG. 2c shows Sanger sequencing chromatograms of the target site in the target gene of wild-type mouse and mutant newborn pups (T113 and T114). As seen, the mutant newborn pups had a stop codon (TAG) introduced by single base substitution (C→T) to the target site.

FIG. 2d shows phenotypes of the eyes of the mutant newborn pups, exhibiting ocular albinism in the mutant mice (T113 and T114). The result implies that the Tyr gene was successfully knocked down by the introduction (electroporation) of RNP of BE3 mRNA and Tyr targeted sgRNA.

### EXAMPLE 13: Assay for Off-Target Effect

In order to assay off-target effects of BE3, potential off-target sites having up to 3-nucleotide mismatches were found in the moue genome by using the Cas-OFFinder (http://www.rgenome.net/cas-offinder/) and genomic DNA isolated from the mutant newborn pups were analyzed using targeted deep sequencing.

sgRNA sequence and primer sequences used in targeted deep sequencing are summarized in Table 3, below.

**[TABLE 3]**

| sgRNA sequence | |
|---|---|
| Dmd | AAGCCAGTTAAAAATTTGTAAGG (SEQ ID NO: 19) |
| Tyr | ACCTCAGTTCCCCTTCAAAGGGG (SEQ ID NO: 35) |

| sgRNA primer for T7 in vitro transcription (5'-3') | |
|---|---|
| Dmd-F | GAA ATT AAT ACG ACT CAC TAT AGA AGC CAG TTA AAA ATT TGT AGT TTT AGA GCT AGA AAT AGC AAG (SEQ ID NO: 53) |
| Tyr-F | GAA ATT AAT ACG ACT CAC TAT AGA CCT CAGTTC CCC TTC AAA GGT TTT AGA GCT AGA AATAGC AAG (SEQ ID NO: 54) |
| R | AAAAAAGCACCGACTCGGTGCCACTTTTTCAAGTTGATAAC GGACTAGCCTTATTTTAACTTGCTATTTCTAGCTCTAAAAC (SEQ ID NO: 55) |

| Targeted deep sequencing primer (5'-3') | |
|---|---|
| Dmd-1st PCR-F | GCT AGA GTA TCA AAC CAA CAT CAT TAC (SEQ ID NO: 56) |
| Dmd-1st PCR-R | TGC TTC CTA TCT CAC CCA TCT (SEQ ID NO: 57) |
| Dmd-2nd/adaptor PCR-F | ACA CTC TTT CCC TAC ACG ACG CTC TTC CGA TCT GCT ACA ACA ATT GGA ACA GAT GAC (SEQ ID NO: 58) |
| Dmd-2nd/adaptor PCR-R | GTG ACT GGA GTT CAG ACG TGT GCT CTT CCG ATC TTC TTC ACT GTA CAG AGC TCA ATG (SEQ ID NO: 59) |
| Tyr-1st PCR-F | TGT ATT GCC TTC TGT GGA GTT (SEQ ID NO: 61) |
| Tyr-1st PCR-R | GGT GTT GAC CCA TTG TTC ATT T (SEQ ID NO: 61) |
| Tyr-2nd/adaptor PCR-F | ACA CTC TTT CCC TAC ACG ACG CTC TTC CGATCT GGA GTT TCC AGA TCT CTG ATG G (SEQ ID NO: 62) |
| Tyr-2nd/adaptor PCR-R | GTG ACT GGA GTT CAG ACG TGT GCT CTT CCGATC TGC ACT GGC AGG TCC TAT TAT (SEQ ID NO: 63) |

The off-target sites identified in the mouse genome and the targeted deep sequencing results are given in Tables 4 (Dmd) and 5 (Tyr) and FIGS. 4 (Dmd) and 5 (Tyr).

**[TABLE 4]**

| Off-target sites for Dmd target site in mouse genome (Dmd-On: on-target site of Dmd; Dmd-OT: off-target site of Dmd) | | | | | | |
|---|---|---|---|---|---|---|
| No. | Gene | | Sequence | Chromoso me | Position | Direct ion |
| *Dmd*-On | *Dmd* | Exon | AAGCCAGTTAAAAATTTG TAAGG (SEQ ID NO:19) | chrX | 83781748 | + |
| *Dmd-*OT1 | *Atp11c* | Intron | AAGCCAGTTAgAAgTTTG TAAGG (SEQ ID NO:20) | chrX | 60354718 | + |
| *Dmd-*OT2 | Intergenic region | - | AAGCaAGTgtAAAATTTGT ATGG (SEQ ID NO:21) | chr3 | 12391596 4 | - |
| *Dmd-*OT3 | *Ptprd* | Intron | AAtaCAGTTAAtAATTTGTA AGG (SEQ ID NO:22) | chr4 | 78045958 | + |
| *Dmd-*OT4 | *Cfh* | Intron | AAGCaAGTaAAAcATTTGT ATGG (SEQ ID NO:23) | chr1 | 13993396 7 | + |
| *Dmd-*OT5 | *Sos1* | Intron | ttGCCAGTTtAAAATTTGTA AGG (SEQ ID NO:24) | chr17 | 80462531 | - |
| *Dmd-*OT6 | Intergenic region | - | AAGCaAtTgAAAAATTTGT ATGG (SEQ ID NO:25) | chr17 | 81921018 | + |
| *Dmd-*OT7 | *Grid2* | Intron | AAGCCAGaaAcAAATTTG TAGGG (SEQ ID NO:26) | chr6 | 64297703 | - |
| *Dmd-*OT8 | Intergenic region | - | AAGCtAGTggAAAATTTGT ACGG (SEQ ID NO:27) | chr6 | 14775958 2 | - |
| *Dmd-*OT9 | Intergenic region | - | AActCAaTTAAAAATTTGT ATGG (SEQ ID NO:28) | chr14 | 26539529 | - |
| *Dmd-*OT10 | *Spag9* | Intron | AAGCCAGagAAtAATTTGT AGGG (SEQ ID NO:29) | chr11 | 94052933 | + |
| *Dmd-*OT11 | *Tle 1* | Intron | AAaCCAGTTAAAtATTTcT AAGG (SEQ ID NO:30) | chr4 | 72159600 | + |
| *Dmd-*OT12 | *RNf114b* | Intron | AAGCCAtTTAAAAATTTGa gTGG (SEQ ID NO:31) | chr13 | 47235657 | - |
| *Dmd-*OT13 | *Tnfrsf21* | Intron | AAcCCAGTTAgAAATTTtT ATGG (SEQ ID NO:32) | chr17 | 43052831 | + |
| *Dmd-*OT14 | *Aak1* | Intron | AAGaCAGaTAAAAATTTG gAGGG (SEQ ID NO:33) | chr6 | 86907351 | - |
| *Dmd-*OT15 | *Gpm6b* | 3' UTR | AgGCCAGaTAAAAATTTG aAGGG (SEQ ID NO:34) | chrX | 16638599 4 | + |

**[TABLE 5]**

| Off-target site for Tyr target site in mouse genome (Tyr-On: on-target site of Tyr; Tyr -OT: off-target site of Tyr) | | | | | | |
|---|---|---|---|---|---|---|
| No. | Gene | | Sequence | Chromosom e | Position | Direct ion |
| *Tyr-*On | *Tyr* | Exon | ACCTCAGTTCCCCTTCAA AGGGG (SEQ ID NO:35) | chr7 | 87493130 | - |
| *Tyr-*OT1 | *Zmat4* | Intron | cCCTCAGTTCCaCTTCAg AGAGG (SEQ ID NO:36) | chr8 | 23975596 | - |
| *Tyr-*OT2 | Intergenic | - | ACCTCAcTTgCCCTTCtAA GTGG (SEQ ID NO:37) | chr8 | 10205994 2 | + |
| *Tyr-*OT3 | *II2* | Intron | ACCTCAGTcCCCCTTtAcA GAGG (SEQ ID NO:38) | chr3 | 37123254 | + |
| *Tyr-*OT4 | Intergenic | - | ACCTCAGTTCCCCTaCAct GGGG (SEQ ID NO:39) | chr3 | 9150768 | - |
| *Tyr-*OT5 | Intergenic | - | ACCTCAGTTCCCCTaCAct GGGG (SEQ ID NO:40) | chr3 | 81773804 | - |
| *Tyr-*OT6 | Intergenic | - | tCCTCAGTTCCCCTTCAct GGGG (SEQ ID NO:41) | chr7 | 11778250 | + |
| *Tyr-*OT7 | Intergenic | - | cCCTCAGTTCCCCTaCAc AGAGG (SEQ ID NO:42) | chr4 | 47766122 | - |
| *Tyr-*OT8 | Intergenic | - | ACCTCAGTTtCCCTTCcAg GAGG (SEQ ID NO:43) | chr4 | 54553337 | - |
| *Tyr-*OT9 | 2810429I04Rik | Intron | cCCTCAGTTCCCCTTCAct GGGG (SEQ ID NO:44) | chr13 | 3491261 | + |
| *Tyr-*OT10 | Intergenic | - | cCCTCAGTTCCCCTaCAc AGGGG (SEQ ID NO:45) | chr13 | 74957186 | + |
| *Tyr-*OT11 | MGP_C57BL6NJ G0001126 | - | cCCTCAGTTCCCCTaCAc AGGGG (SEQ ID NO:46) | chr2 | 79031825 | + |
| *Tyr-*OT12 | *Rai14* | Intron | ACCTCAGTTtCCCcTCAA AaTGG (SEQ ID NO:47) | chr15 | 10596653 | + |
| *Tyr-*OT13 | Intergenic | - | ACCTCAGTTgtCCTTCAAA cAGG (SEQ ID NO:48) | chr6 | 10738434 8 | - |
| *Tyr-*OT14 | D6Ertd474e | Intron | cCCTCAGTTCCCCTaCAAt GGGG (SEQ ID NO:49) | chr6 | 14324745 6 | - |
| *Tyr-*OT15 | Intergenic | - | AaCTCtGTTCCCCTTCtAA GTGG (SEQ ID NO:50) | chr18 | 23151245 | + |
| *Tyr-*OT16 | Intergenic | - | AtCTCAGTTtCCCTTCAcA GGGG (SEQ ID NO:51) | chr11 | 71708976 | - |
| *Tyr-*OT17 | *Foxk2* | Intron | ACCTtAGTTCCCtTTCAAA cTGG (SEQ ID NO:52) | chr11 | 12127164 9 | + |

(In Tables 5 and 6, mismatched nucleotides in off-target sites with the on-target sequence are represented in lower cases: NGG at the 3' end accounts for the PAM sequence)

As can be seen in Tables 4 and 5 and FIGS. 4 and 5, the on-target sites used in this assay were observed not to induce significant off-target mutation, demonstrating that the BE3 system targeting the on-target sites is significantly specific for the targets *in vivo.*

## Claims

1. A base editing method for a non-human mammalian embryonic cell, the method comprising:
directly injecting 1) a ribonucleoprotein complex or mixture, the ribonucleoprotein complex or mixture comprising a cytidine deaminase, an RNA-guided nuclease, and a guide RNA, or 2) a mixture of a cytidine deaminase-encoding mRNA, an RNA-guided nuclease-encoding mRNA, and a guide RNA,
into the non-human mammalian embryonic cell without using a recombinant vector.

2. The base editing method of claim 1, wherein the RNA-guided nuclease is a Cas9 protein or a Cpf1 protein; or
the RNA-guided nuclease is Cas9 nickase, a catalytically deficient Cas9 protein, or a Cas9 protein that recognizes a PAM sequence different from a wild-type Cas9 protein.

3. The base editing method of claim 1 or 2, wherein the RNA-guided nuclease comprising an amino acid sequence of *Streptococcus pyogenes-derived* Cas9 protein wherein the following amino acid residues are substituted with amino acid residues different from wild-type amino acid residues:
(1) D10, H840, or D10 and H840;
(2) at least one selected from the group consisting of D1135, R1335, T1337; or
(3) both of (1) and (2) amino acid residues.

4. The base editing method of any one of claims 1 to 3, wherein the guide RNA is a dual RNA or single guide RNA (sgRNA) comprising CRISPR RNA (crRNA) and trans-activating crRNA (tracrRNA).

5. The base editing method of any one of claims 1 to 4, wherein the cytidine deaminase is APOBEC (apolipoprotein B mRNA editing enzyme, catalytic polypeptide-like), AID (activation-induced cytidine deaminase), tadA (tRNA-specific adenosine deaminase), or a combination thereof.

6. The base editing method of any one of claims 1 to 5, wherein
the base editing composition for a mammalian cell comprises a cytidine deaminase-encoding mRNA, an RNA-guided nuclease-encoding mRNA, and a guide RNA, or
the base editing composition for a mammalian cell comprises a ribonucleoprotein in which a cytidine deaminase, an RNA-guided nuclease, and a guide RNA form together a complex.

7. The base editing method of any one of claims 1 to 6, further comprising a uracil DNA a glycosylase inhibitor (UGI) or a coding gene therefor, a nuclear localization sequence (NLS) or a coding gene therefor, or all of them.

8. The base editing method of any one of claims 1 to 7, wherein the injecting step is conducted by microinjection or electroporation.

## Patentansprüche

1. Baseneditierungsverfahren für eine embryonale Zelle eines nicht-menschlichen Säugetiers, wobei das Verfahren Folgendes beinhaltet:
direktes Injizieren 1) eines Ribonukleoprotein-Komplexes oder -Gemischs, wobei der/das Ribonukleoprotein-Komplex oder -Gemisch eine Cytidin-Desaminase, eine RNA-geführte Nuklease und eine Guide-RNA umfasst, oder 2) eines Gemischs aus einer Cytidin-Desaminase-kodierenden mRNA, einer RNA-geführten Nuklease-kodierenden mRNA und einer Guide-RNA,
in die nicht-menschliche embryonale Säugetierzelle, ohne einen rekombinanten Vektor zu verwenden.

2. Baseneditierungsverfahren nach Anspruch 1, wobei die RNA-geführte Nuklease ein Cas9-Protein oder ein Cpf1-Protein ist; oder
die RNA-geführte Nuklease eine Cas9-Nickase, ein katalytisch defizientes Cas9-Protein oder ein Cas9-Protein ist, das eine PAM-Sequenz erkennt, die sich von einem Wildtyp-Cas9-Protein unterscheidet.

3. Baseneditierungsverfahren nach Anspruch 1 oder 2, wobei die RNA-geführte Nuklease eine Aminosäuresequenz eines von *Streptococcus pyogenes* abgeleiteten Cas9-Proteins umfasst, in der die folgenden Aminosäurereste durch Aminosäurereste ersetzt sind, die sich von Wildtyp-Aminosäureresten unterscheiden:
(1) D10, H840, oder D10 und H840;
(2) mindestens einer ausgewählt aus der Gruppe bestehend aus D1135, R1335, T1337; oder
(3) beide Aminosäurereste aus (1) und (2).

4. Baseneditierungsverfahren nach einem der Ansprüche 1 bis 3, wobei die Guide-RNA eine duale RNA oder eine einzelne Guide-RNA (sgRNA) ist, die CRISPR-RNA (crRNA) und trans-aktivierende crRNA (tracrRNA) umfasst.

5. Baseneditierungsverfahren nach einem der Ansprüche 1 bis 4, wobei die Cytidin-Desaminase APOBEC (Apolipoprotein B mRNA-Editierenzym, katalytisches Polypeptid-ähnlich), AID (Aktivierungs-induzierte Cytidin-Desaminase), tada (tRNAspezifische Adenosin-Desaminase) oder eine Kombination davon ist.

6. Baseneditierungsverfahren nach einem der Ansprüche 1 bis 5, wobei
die Baseneditierungszusammensetzung für eine Säugetierzelle eine Cytidin-Deaminase-kodierende mRNA, eine RNA-geführte Nuklease-kodierende mRNA und eine Guide-RNA umfasst, oder
die Baseneditierungszusammensetzung für eine Säugetierzelle ein Ribonukleoprotein umfasst, in dem eine Cytidin-Deaminase, eine RNA-geführte Nuklease und eine Guide-RNA zusammen einen Komplex bilden.

7. Baseneditierungsverfahren nach einem der Ansprüche 1 bis 6, das ferner einen Uracil-DNA-Glykosylase-Inhibitor (UGI) oder ein kodierendes Gen dafür, eine Nuklear-Lokalisierungssequenz (NLS) oder ein kodierendes Gen dafür oder alle davon beinhaltet.

8. Baseneditierungsverfahren nach einem der Ansprüche 1 bis 7, wobei der Injektionsschritt durch Mikroinjektion oder Elektroporation durchgeführt wird.

## Revendications

1. Procédé d'édition de bases pour une cellule embryonnaire de mammifère non humain, le procédé comprenant :
l'injection directe 1) d'un complexe ou mélange ribonucléoprotéique, le complexe ou mélange ribonucléoprotéique comprenant une cytidine désaminase, une nucléase guidée par ARN et un ARN guide, ou 2) d'un mélange d'un ARNm codant pour la cytidine désaminase, d'un ARNm codant pour la nucléase guidée par ARN et d'un ARN guide,
dans la cellule embryonnaire de mammifère non humain sans utiliser de vecteur recombinant.

2. Procédé d'édition de bases selon la revendication 1, dans lequel la nucléase guidée par ARN est une protéine Cas9 ou une protéine Cpf1 ; ou
la nucléase guidée par ARN est Cas9 nickase, une protéine Cas9 déficiente catalytiquement, ou une protéine Cas9 qui reconnaît une séquence PAM différente d'une protéine Cas9 de type sauvage.

3. Procédé d'édition de bases selon la revendication 1 ou 2, dans lequel la nucléase guidée par ARN comprend une séquence d'acides aminés de la protéine Cas9 dérivée de *Streptococcus pyogenes* dans lequel les résidus d'acides aminés suivants sont substitués par des résidus d'acides aminés différents de résidus d'acides aminés de type sauvage :
(1) D10, H840 ou D10 et H840 ;
(2) au moins un résidu sélectionné dans le groupe constitué par D1135, R1335, T1337 ; ou
(3) les deux (1) et (2) résidus d'acides aminés.

4. Procédé d'édition de bases selon l'une quelconque des revendications 1 à 3, dans lequel l'ARN guide est un ARN double ou un ARN guide simple (ARNsg) comprenant l'ARN CRISPR (ARNcr) et l'ARNcr transactivateur (ARNtracr).

5. Procédé d'édition de bases selon l'une quelconque des revendications 1 à 4, dans lequel la cytidine désaminase est APOBEC (enzyme d'édition d'ARNm de l'apolipoprotéine B, se comportant comme un polypeptide catalytique), AID (cytidine désaminase induite par activation), tadA (adénosine désaminase spécifique de l'ARNt), ou une combinaison de ceux-ci.

6. Procédé d'édition de bases selon l'une quelconque des revendications 1 à 5, dans lequel
la composition d'édition de bases pour une cellule de mammifère comprend un ARNm codant pour la cytidine désaminase, un ARNm codant pour une nucléase guidée par ARN et un ARN guide, ou
la composition d'édition de bases pour une cellule de mammifère comprend une ribonucléoprotéine dans laquelle une cytidine désaminase, une nucléase guidée par ARN et un ARN guide forment ensemble un complexe.

7. Procédé d'édition de bases selon l'une quelconque des revendications 1 à 6, comprenant en outre un inhibiteur de l'uracile-ADN-glycolase (UGI) ou un gène codant pour celui-ci, une séquence de localisation nucléaire (NLS) ou un gène codant pour celle-ci, ou tous ceux-ci.

8. Procédé d'édition de bases selon l'une quelconque des revendications 1 à 7, dans lequel l'étape d'injection est réalisée par micro-injection ou électroporation.
